# EUROPEAN PATENT APPLICATION

(11) **EP 3 777 871 A1**
(43) Date of publication of application: **17.02.2021**
(21) Application number: 19781599.6
(22) Date of filing: 25.03.2019
(51) Int. Cl.: A61K 36/537, A61K 36/258, A61K 36/481, A61K 31/7048, A61P 9/10

(54) **TRADITIONAL CHINESE MEDICINE COMPOSITION FOR PREVENTING AND/OR TREATING ISCHEMIC REPERFUSION INJURY**

(30) Priority: 04.04.2018 CN 201810299213
(71) Applicant: Tasly Pharmaceutical Group Co., Ltd., Tianjin 300410 (CN)
(72) Inventor: HAN, Jingyan, Tianjin 300410 (CN); CHEN, Qingfang, Tianjin 300410 (CN); HUANG, Dandan, Tianjin 300410 (CN); MA, Xiaohui, Tianjin 300410 (CN); HE, Yi, Tianjin 300410 (CN); ZHOU, Shuiping, Tianjin 300410 (CN)
(74) Representative: Gulde & Partner
(86) International application number: PCT/CN2019/079420
(87) International publication number: WO 2019/192339

(57) **Abstract**

The present invention discloses a traditional Chinese medicinal composition for preventing and/or treating ischemia reperfusion injury; the traditional Chinese medicinal composition consists of salvianolic acids, *Panax Notoginseng* saponins and total saponins of *Astragalus.*

## Description

### Technical Field

The present invention relates to the field of traditional Chinese medicine, and in particular to a traditional Chinese medicinal composition for preventing and/or treating ischemic reperfusion injury.

### Background Art

In 1966, Jennings proposed the concept of ischemic reperfusion injury for the first time: when histocyte is supplied by blood once again after suffering low-perfusion ischemia, the reperfusion of blood flow aggravates the ischemic injury rather than relieving or recovering the ischemic lesion of cells. It is a common phenomenon on a body of higher animal caused by ischemic reperfusion. Ischemic reperfusion injury may occur in a body after receiving a cardiac surgery, coronary artery bypass surgery, reperfusion after infarction of visceral blood flow, organ transplantation and correction of low perfusion in shock organs. The degree of injury is closely related to ischemic time, circulation of collateral blood vessels, oxygen demand, conditions of reperfusion, etc.

The treatment of ischemic diseases gives priority to the recovery of blood perfusion, which aims at relieving anoxia of tissues and undersupply of nutrients, thus holding back the development of ischemic injury or facilitating its recovery.

In recent years, with the improvement of shock therapy, and establishment, popularization and application of off-pump coronary artery bypass (OPCAB), thrombolytic therapy, percutaneous transluminal coronary angioplasty (PTCA), extracorporeal circulation of cardiac surgery, cardiopulmonary-cerebral resuscitation, replantation of severed limbs, organ transplantation and other methods, a lot of tissues and organs have been perfused by blood flow once again after suffering ischemia.

A large number of clinical practices have proved that such kind of therapy achieves good effect. Therefore, the recovery of blood perfusion has become the basic principle to treat ischemic diseases.

Correspondingly, medicaments for treating ischemia reperfusion injury include free-radical scavengers, antioxidants, calcium antagonists, channel inhibitors, anti-inflammatory medicaments, etc. currently.

Salvianolic acids, a salvianolic acid injection as its dosage form used clinically, plays the role of activating blood circulation, dispersing blood stasis and dredging the channels. The injection is clinically used for treating coronary heart disease stable angina pectoris, classified into grades I and II. There are mild and moderate symptoms of angina pectoris. By TCM syndrome differentiation, the patient suffering from cariac blood stasis syndrome shows chest pain, chest distress and palpitation.

Panax notoginsenosides (*Panax Notoginseng* saponins), the Xuesaitong injection as its dosage form used clinically, has the following pharmacological action of: expanding coronary and peripheral vessels, reducing peripheral resistance, slowing down heart rate, lowering myocardial oxygen consumption, increasing myocardial perfusion, adding cerebral blood flow and improving myocardial and cerebral ischemia to some extent; significantly inhibiting platelet aggregation, reducing blood viscosity and inhibiting thrombosis. Moreover, the injection has multiple effects, such as reduction of blood fat, antifatigue, anti-hypoxia, improvement and enhancement of macrophage. The Xuesaitong injection is mainly clinically used for treating cerebral vascular sequela, occlusion of venae centralis retinae, hyphema, etc.

Total saponins of *Astragalus* are clinically used for anti-thrombosis, and can enhance immunity, tonifying middle-Jiao and Qi.

Currently, there is no report on the combination of salvianolic acids, *Panax Notoginseng* saponins and total saponins of *Astragalus.*

### Summary of the Invention

The objective of the present invention is to provide a traditional Chinese medicinal composition for preventing and/or treating ischemic reperfusion injury. The traditional Chinese medicinal composition consists of salvianolic acid, *Panax Notoginseng* saponins and total saponins of *Astragalus.*

The weight ratio of salvianolic acids to *Panax Notoginseng* saponins and total saponins of *Astragalus* is (1-16): (1-16): (1-16).

Preferably, in the traditional Chinese medicinal composition, the weight ratio of salvianolic acids to *Panax Notoginseng* saponins and total saponins of *Astragalus* is (4-16): (1-8): (1-16).

Further preferably, in the traditional Chinese medicinal composition, the weight ratio of salvianolic acids to *Panax Notoginseng* saponins and total saponins of *Astragalus* is (4-16): (1-4): (1-16).

Further preferably, in the traditional Chinese medicinal composition, the weight ratio of salvianolic acids to *Panax Notoginseng* saponins and total saponins of *Astragalus* is (4-8): (1-4): (1-16), or (8-16): (1-4): (1-16).

More preferably, in the traditional Chinese medicinal composition, the weight ratio of salvianolic acids, *Panax Notoginseng* saponins and total saponins of *Astragalus* is (4-8): 1: (5-16), or (4-8): (1-2): (1-5).

Most preferably, the weight ratio of salvianolic acid (a) to *Panax Notoginseng* saponins (b) and total saponins of *Astragalus* (c) is 4: 1: 5.

### In the ingredients of the present invention:

Salvianolic acids are extractive comprising 40-95% of salvianolic acid B, 20-95% of salvianic acid A sodium, 3-15% of rosmarinic acid, 2-10% of alkannic acid and 0.2-2.2% of salvianolic acid E by weight percentage.

Salvianolic acids may be prepared by the following method: Salvia miltiorrhiza slices are extracted by alcohol at a concentration of 20% to 90%, and then the extracting solution is concentrated to be free of alcohol; the extractive is processed through polyamide chromatography, and washed by water or ethyl alcohol at a concentration of 30% below to remove impurities, and then eluted by alcohol at a concentration of 30% to 95% or sodium bicarbonate at a concentration of 0.05% to 0.3% or sodium carbonate at a concentration of 0.01% to 0.3%; eluent is collected and regulated pH=1-5.5, passing through a low-polar or non-polar macroporous resin, then washed by water or alcohol at a concentration of 30% below to remove impurities, and eluted by 30-95% of alcohol to collect eluent; the eluent is collected, concentrated and dried to obtain the final product.

Panax *Notoginseng saponins* are extractive comprising 5-20% of notoginsenoside R1, not less than 20% of ginsenoside Rb1, 3-15% of ginsenoside Rd, not less than 30% of ginsenoside Rg1 and not less than 2% of ginsenoside Re; total content of the five saponins is not less than 80%.

*Panax Notoginseng* saponins may be prepared by the following method: Panax notoginseng herbs are taken and crushed into coarse particles, added 3-10 times of ethyl alcohol at a concentration of 20% to 80% for reflux extraction twice, 2-5 hours each time, the extracting solution is blended, concentrated by pressure reduction to be free of alcohol, then centrifuged; supernatant is enriched by a macroreticular resin, accharides and partial pigments are eluted by water with 3-10 times of column volume, and then continuously eluted by 30-70% of ethyl alcohol with 5-10 times of column volume to collect alcohol eluent, and the eluent is concentrated by pressure reduction to a dry powder, thus obtaining *Panax Notoginseng* saponins.

Total saponins of *Astragalus* are extractive, account for 20-100% of a weight percentage, and the content of astragaloside is within the range of 20% to 95%.

Total saponins of *Astragalus* may be exacted by the following method: *Radix Astragali* herbal slices are taken and crushed into coarse particles, added 3-10 times of alcohol at a concentration of 20 % to 70% (containing 0.1-0.5% of sodium bicarbonate) for reflux extraction twice, 2-5 hours each time; the extracting solution is blended, concentrated by pressure reduction to 2-5 times of volume of the herbs and centrifuged; supernatant is enriched by a macroreticular resin, and washed by 2-8 times of volume of NaOH solution at a concentration of 0.1% to 1% firstly, then eluted by 2-8 times of volume of alcohol at a concentration of 20% to 60%, and finally, eluted by 2-8 times of volume of alcohol at a concentration of 75% to 95% to collect an alcohol eluent at a concentration of 70% to 95%. The eluent is decolorized by a macroporous resin and eluted by 2-5 times of column volume of alcohol at a concentration of 70% to 95% to collect effluent and eluent; effluent and eluent are concentrated by pressure reduction to a small volume, and crystal precipitates out solid, then the solid is collected for drying to obtain total saponins of *Astragalus.*

The other objective of the present invention is to provide a formulation comprising the traditional Chinese medicinal composition, and the formulation consists of the traditional Chinese medicinal composition and a pharmaceutically acceptable carrier.

The pharmaceutically acceptable carrier refers to a conventional carrier in the field of pharmacy, and is selected from one or more of a group consisting of a filler, an adhesive, a disintegrating agent, a lubricant, a solubilizer, a suspending agent, a wetting agent, a pigment, a solvent, a surfactant or a corrigent.
The filler is selected from starch, pregelatinized starch, dextrin, glucose, sucrose, lactose, lactitol, microcrystalline cellulose, mannitol, sorbitol or xylitol;
The adhesive is selected from sodium carboxymethylcellulose, hydroxypropyl methyl cellulose, ethyecellulose, povidone, starch slurry, sucrose, powdered sugar, mucilage, gelatin or polyethylene glycol;
The disintegrating agent is selected from croscarmellose sodium, polyvinylpolypyrrolidone, polyvinylpolypyrrolidone, low-substituted hydroxypropyl cellulose, sodium carboxymethyl starch or starch;
The lubricant is selected from magnesium stearate, talcum powder, superfine silica powder, PEG4000, PEG6000 or sodium laurylsulfate;
The solubilizer is selected from sodium hydroxide, potassium hydroxide, sodium bicarbonate, meglumine, L-lysine or L-arginine;
The suspending agent is selected from superfine silica powder, beewax, cellulose or solid polyethylene glycol;
The wetting agent is selected from glycerinum, Tween-80, ethyoxyl hydrogenated castor oil or lecithin;
The solvent is selected from ethyl alcohol, liquid polyethylene glycol, isopropanol, Tween-80, glycerinum, propylene glycol or vegetable oil; the vegetable oil is selected from soybean oil, castor oil, peanut oil, blend oil, etc;
The surfactant is selected from sodium dodecyl benzene sulfonate, stearic acid, polyethylene oxide-polypropylene oxide copolymer, fatty acid sorbitan or polysorbate (Tween), etc;
The corrigent is selected from Aspartame, sucralose, essence, Steviosin, Acesulfame, citric acid or sodium saccharin.

The other objective of the present invention is to provide uses of the traditional Chinese medicinal composition.

One is use of the traditional Chinese medicinal composition of the present invention in treating and/or preventing ischemia reperfusion injury. The ischemia reperfusion injury of the present invention includes but not limited to, cerebral ischemia reperfusion injury, myocardial ischemia reperfusion injury, renal ischemia reperfusion injury, lower limb ischemia reperfusion injury, ischemia reperfusion injury of spinal cord, retina ischemia reperfusion injury, flap ischemia reperfusion injury, etc.

The ischemia reperfusion injury of the present invention includes but not limited to, ischemia reperfusion injury caused by thrombolysis, ischemia reperfusion injury caused by OPCAB, ischemia reperfusion injury caused by PTCA, ischemia reperfusion injury caused by extracorporeal circulation of cardiac surgery, ischemia reperfusion injury caused by cardiopulmonary-cerebral resuscitation, ischemia reperfusion injury caused by replantation of severed limbs and organ transplantation, etc.

Another one is to combine the traditional Chinese medicinal composition of the present invention with tissue-type plasminogen activator (tPA) for thrombolysis; in the second use, the traditional Chinese medicinal composition and tPA are blended according to the weight ratio of 10: (5-20).

The traditional Chinese medicinal composition may ease exudation and hemorrhage caused by tPA, increase survival rate and reduce nerve injury.

The traditional Chinese medicinal composition of the present invention has the following advantages:
The traditional Chinese medicinal composition of the present invention can be used for treating and/preventing ischemia reperfusion injury and combined with tPA for thrombolysis, and it has good synergy effect and less side effects.

### Brief Description of the Drawings

FIG.1A shows a TTC staining image of brain tissue slices in each group of rats after reperfused for 24h;
FIG.1B is a statistical graph showing TTC infarct size of mice in different groups;
FIGS. 2A and 2B respectively show results of Modified Neurological Severity Score in each group of rats after reperfused for 3h and 24h;
FIG.3 shows a dynamic change in the thrombus of arteria carotis communis in each group of mice Initial value is a base value before FeCl3 stimulation. 10 minutes denote 10 min after the beginning of FeCl3 stimulation (FeCl3 filter paper is wrapped on arteria carotis communis for 3 minutes, and then removed). 4.5 hours denote 4.5 h after the beginning of wrapping arteria carotis communis by FeCl3 filter paper, namely, the start time of administration. 5.5 hours denote 1 h after administration. 6.5 hours denote 2 h after administration; 24 hours denote the 24 h after administration;
FIG.4 shows an image of brain surface blood perfusion of mice detected by a Laser Doppler Flowmetry. Initial value is a base value before FeCl3 stimulation. 10 minutes denote 10 min after the beginning of FeCl3 stimulation (FeCl3 filter paper is wrapped on arteria carotis communis for 3 minutes, and then removed). 4.5 hours denote 4.5 h after the beginning of wrapping arteria carotis communis by FeCl3 filter paper, namely, the start time of administration. 5.5 hours denote 1 hour after administration. 6.5 hours denote 2 h after administration; 24 hours denote the 24 h after administration;
FIG.5 denotes Evans Blueleakage in brain tissue 24 hours after administration, in the figure, when * is compared with sham-operated group, p<0.05; when # is compared with tPA thrombolysis group, p<0.05; N=6;
FIG.6 shows vascular permeability of brain surface postcapillary venule in ischemic penumbra; in the figure, when * is compared with sham-operated group, p<0.05; when # is compared with tPA thrombolysis group, p<0.05; N≤6;
FIG.7 shows a change of cerebral perivascular edema, opening number of microvessels, and dry/wet weight ratio 24 hours after administration; in the figure, when * is compared with sham-operated group, p<0.05; when # is compared with tPA thrombolysis group, p<0.05; N≤6;
FIG.8 shows a change of endotheliocyte connexin of brain microvessels 24 hours after administration and transmission electron microscope (TEM) images of gap-junctions of cerebrovascular endothelial cell; arrows denote tight junctions (TJ) of vascular endothelial cells; Western blotting and quantitative statistics on ZO-1, VE-cadherin, occluding and JAM of right hemisphere ischemic penumbra cortex. In the figure, when * is compared with sham-operated group, p<0.05; when † is compared with tPA thrombolysis group, p<0.05. For electronic microscope, N=3, and N≥6 for the rest;
FIG.9 denotes a change of connexins of endothelial cells cultured in vitro after undergoing hypoxia/reoxygenation, Western blotting representing images and statistics of tight-junction proteins Claudin-5, JAM-1 and an adherent junction VE-cadherin among cerebral microvascular endothelial cells after deprived of oxygen for 4.5 hours, then reoxygenated and supplied tPA and/or T541. Hypoxia/reoxygenation, H/R. In the figure: when * is compared with normal control, p<0.05 vs; when # is compared with model group, p<0.05. N≥4;

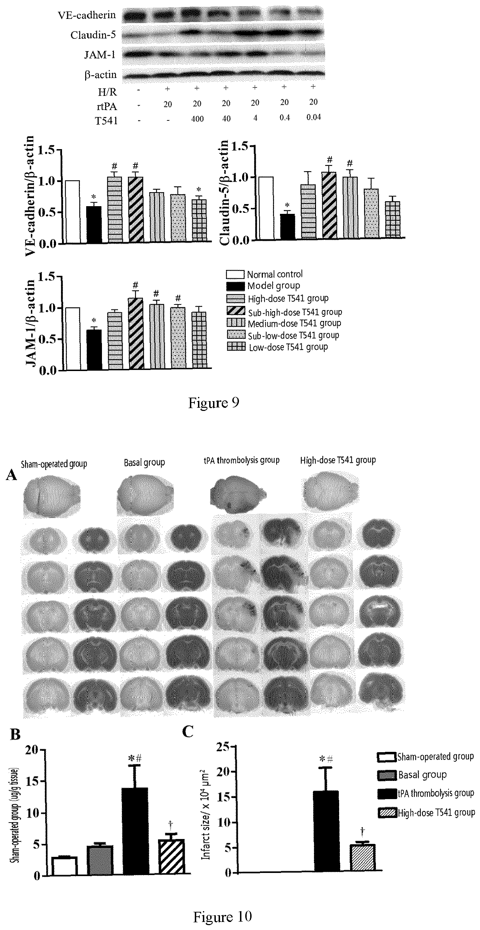

FIG.10 shows a condition of cerebral hemorrhage, and representing diagrams of cerebral hemorrhage and cerebral infarction 24 hours after administration. Mice brain tissues are taken and cut into 1 mm thickness of slices whose bleeding conditions are shoot, and then the slices are rapidly stained by a TTC dye liquor to record the infarct size. FIG.A shows representing diagrams of cerebral hemorrhage and cerebral infarction 24 hours after administration; FIG.B is a bar graph showing hemoglobin in each group of right hemisphere tissues detected by a hemoglobin spectrophotometry cassete. FIG.C denotes statistics on infarct size of mice in each group. In the figure, when * is compared with sham-operated group, p<0.05; when # is compared with basal group, p<0.05; when † is compared with tPA thrombolysis group, p<0.05. N≥6;
FIG.11 shows a change of basement membrane and related proteins of cerebral ischaemic cortex 24 hours after administration; FIG.A: scanning electron microscope shows a change of basement membrane of cerebral cortex vessels, indicated by white arrows. Basement membrane, BM. N=3.
FIG.B: Western blotting shows the expression quantity of Collagen IV and Laminin in the context of right cerebral ischemic penumbra 24 hours after administration. FIGS.C-D show Western blotting statistics of Collagen IV and Laminin. In the figure, when * is compared with sham-operated group, p<0.05; when # is compared with basal group, p<0.05; when † is compared with tPA thrombolysis group, p<0.05. N= 6;

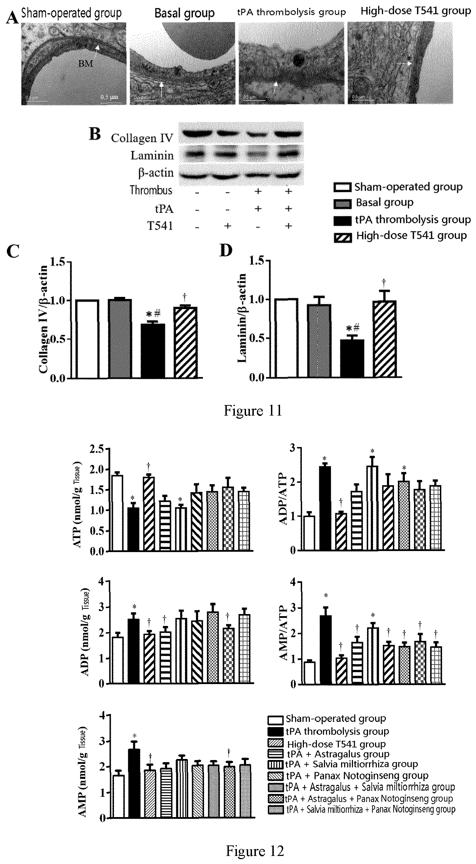

FIG. 12 shows a change of energy of brain tissues 24 hours after administration; in the figure, when * is compared with sham-operated group, p<0.05; when † is compared with tPA thrombolysis group, p<0.05; N= 6-7;

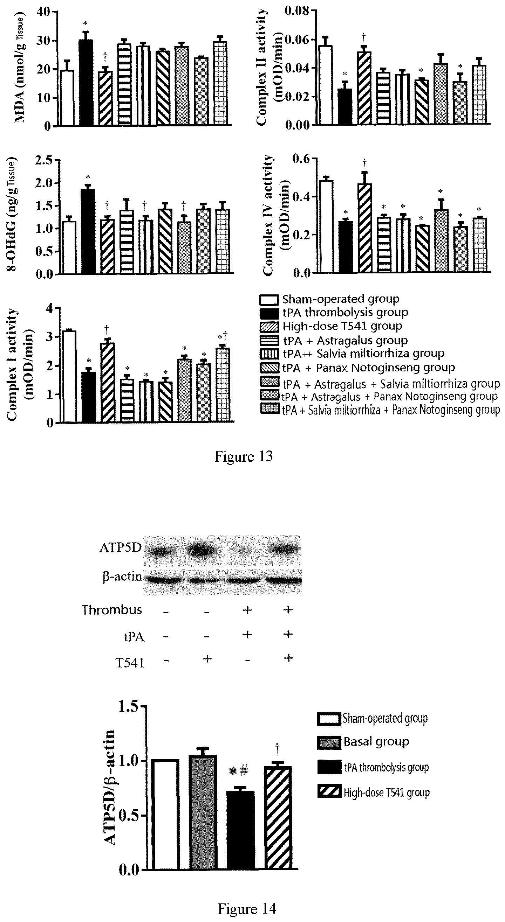

FIG.13 shows oxidative stress injury of brain tissues 24 hours after administration; in the figure, when * is compared with sham-operated group, p<0.05; when † is compared with tPA thrombolysis group, p<0.05. N≥6;
FIG.14 shows a change of ATP5D in brain tissues 24 hours after administration; in the figure, when * is compared with sham-operated group, p<0.05; when # is compared with basal group, p<0.05; when † is compared with tPA thrombolysis group, p<0.05. N= 6;

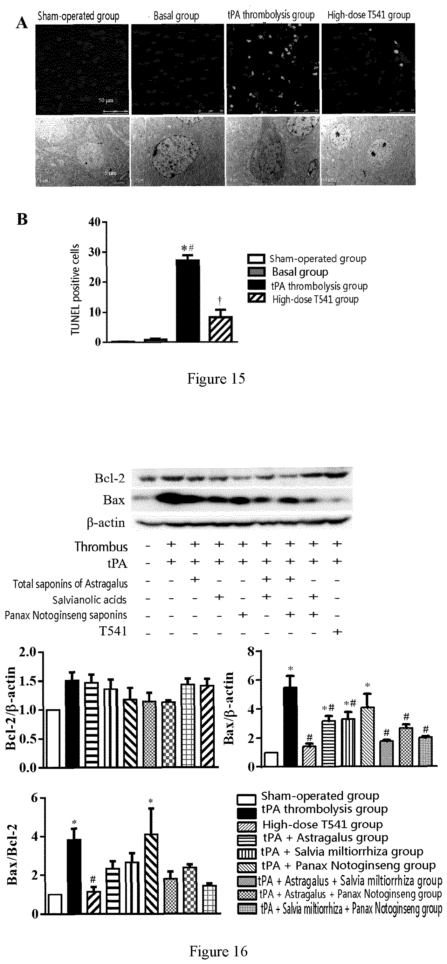

FIG.15 shows apoptosis staining of brain tissues 24 hours after administration; in FIG.A, line 1 shows TUNEL staining of the cerebral cortex in penumbra field 24 hours after administration. Line 2 shows a TEM representing diagram of neuronal apoptosis of the cerebral cortex in penumbra field 24 hours after administration. FIG.B denotes statistics of TUNEL positive cell counting. In the figure, when * is compared with sham-operated group, p<0.05; when # is compared with basal group, p<0.05; when † is compared with tPA thrombolysis group, p<0.05. N= 6;
FIG.16 shows apoptosis of brain tissues 24 hours after administration; in the figure, when * is compared with sham-operated group, p<0.05; when # is compared with tPA thrombolysis group, p<0.05. N= 6;

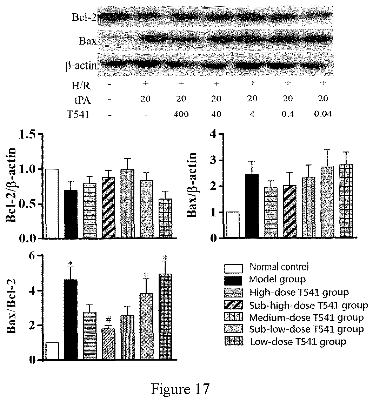

FIG.17 shows apoptosis of endothelial cells cultured in vitro after undergoing hypoxia/reoxygenation; in the figure, when * is compared with normal control, p<0.05; when # is compared with model group, p<0.05. N= 6.

### Detailed Description of the Invention

### Embodiment 1: Traditional Chinese medicinal composition

The weight ratio of salvianolic acids to *Panax Notoginseng* saponins and total saponins of *Astragalus* is 1:2:4.

### Embodiment 2: Traditional Chinese medicinal composition

The weight ratio of salvianolic acids to *Panax Notoginseng* saponins and total saponins of *Astragalus* is 1:4:16.

### Embodiment 3: Traditional Chinese medicinal composition

The weight ratio of salvianolic acids to *Panax Notoginseng* saponins and total saponins of *Astragalus* is 2:16:1.

### Embodiment 4: Traditional Chinese medicinal composition

The weight ratio of salvianolic acids to *Panax Notoginseng* saponins and total saponins of *Astragalus* is 8:1:16.

### Embodiment 5: Traditional Chinese medicinal composition

The weight ratio of salvianolic acids to *Panax Notoginseng* saponins and total saponins of Astragalus is 16:4:1.

### Embodiment 6: Traditional Chinese medicinal composition

The weight ratio of salvianolic acids to *Panax Notoginseng* saponins and total saponins of *Astragalus* is 16:8:4.

### Embodiment 7: Traditional Chinese medicinal composition

The weight ratio of salvianolic acids to *Panax Notoginseng* saponins and total saponins of *Astragalus* is 4:1:5.

### Example 1: Screening experiment on the ratio of salvianolic acids to Panax Notoginseng saponins and total saponins of Astragalus

The three kinds of ingredients were evenly designed to obtain the former 6 groups of table 1, and the rest one group was calculated according to the expected experimental result; there were seven dose groups (ratio) in total:

**Table 1: Proportions of salvianolic acids, Panax Notoginseng saponins and total saponins of Astragalus**

| **Group** | **Salvianolic acids (a)** | ***Panax Notoginseng* saponins (b)** | **Total saponins of *Astragalus* (c)** |
|---|---|---|---|
| 1 | 1 | 2 | 4 |
| 2 | 1 | 4 | 16 |
| 3 | 2 | 16 | 1 |
| 4 | 8 | 1 | 16 |
| 5 | 16 | 4 | 1 |
| 6 | 4 | 2 | 1 |
| 7 | 4 | 1 | 5 |

The effects of the 7 groups were verified by experiments. The total dose of each group was 20 mg/kg.

### 1. Experiment method

### 1.1 Experiment animal

90 270 g-290 g Spragu-Dawley male rats were purchased from Animal Center of the Peking University Health Science Center with the certificate No.: SCXK (Beijing) 2006-0008. Rats were fed with free access to food and water under the conditions of 12h illumination/darkness alternation at 22 ± 2□ and 40% ± 5%. Rats were fasted 12 hours before the experiment but free access to drinking water.

### 1.2 Model building

A suture method was used to cause middle cerebral artery occlusion (MCAO) of rats, thus building I/R MCA models specifically as follows: Rats were anesthetized (intraperitoneal injection) by a compound anesthetic 5 minutes before experiment, and fixed in a supine position, unhaired in the middle part of the neck, sterilized by 75% ethyl alcohol, incised in the middle of the neck (about 3 cm length); muscle and anadesma were separated among the inner edge of sternocleidomastoid, and then arteria carotis communis, external carotid artery and internal carotid were carefully separated away from thyroid glands at both sides of trachea and parathyroid glands above the outside of the trachea. The proximal part of external carotid artery was tied by 6-0 surgical suture; external carotid artery and branches were electrocoagulated. Internal carotid and arteria carotis communis were clipped by a micro-artery clamp for the moment, and an incision was cut on the external carotid artery by microscissors; a thread (diameter= 0.38 mm) whose ends were wrapped by silica gel was slowly inserted into the start point where internal carotid flows to middle cerebral artery through external carotid artery, thus blocking the blood supply of middle cerebral artery. The entry length was about 1.8 cm-2.2 cm away from the crotch of arteria carotis communis. 90 minutes later, the thread was pulled out and skin was sutured to build a rat cerebral I/R model.

After modeling, the traditional Chinese medicinal composition was administrated to each experimental group (10 rats per group) according to different proportions in the table above. Same operation was used in the sham-operated group excepting for the insertion of thread. Anal temperature of rats were kept within (37.0 ± 0.5□) in the whole surgical procedure, and kept continuously by a heating blanket after surgery till the recovery of activity. 24 hours later, rats were anesthetized again and killed for sampling.

### 1.3 Staining of cerebral infarction region

Cerebral infarction of rats after I/R injury was detected by a TTC (2,3,5-triphenyltetrazolium chloride) staining method. The specific steps are as follows: rats were reperfused for 24 hours to take out the brain of each group of rat; the brain was cut into 5 slices on a brain localizer from front to back, and put to 2% TTC for incubation for 15 minutes at 37□, finally, TTC staining was conducted. Non-infarct region was stained red and infarct region was not stained and still white.

Brain TTC staining images were shoot and IamgeJ (Bethesda, MD, USA) was used to calculate a percentage of TTC staining infarct size in the total area of brain, and evaluate the degree of cerebral infarction.

### 1.4 Neurological scoring

Neurological scoring was conducted to each group of rats after reperfused for 3 hours and 24 hours based upon scoring standards (18 scores), as shown in table 2:

**Table 2: Neurological scoring**

| Test Scoring standards | Scores |
|---|---|
| Tail lifting test | 3 |
| Fore limbs bend | 1 |
| Hind legs bend | 1 |
| The angle that head deviated the vertical axis within 30 s was > 10°1 | 1 |
| Place rats on a floor (normal value=0; maximum value=3) | 3 |
| Walk normally | 0 |
| Incapable of walking along the horizontal line | 1 |
| Turn around towards paresis side | 2 |
| topple and fall towards paresis side | 3 |
| Feeling test | 2 |
| Shelf test (visual and touch tests) | 1 |
| Proprioception test (deep sensation, press rats' paws towards the edge of a desk to stimulate limbs and muscle | 2 |
| Beam balance test (normal value=0; maximum value=6) | 6 |
| Stable equilibrium posture | 0 |
| Clutch the edge of the balance beam | 1 |
| Hug the balance beam, and one limb drops from the balance beam | 2 |
| Hug the balance beam, and two limbs drop from the balance beam or rotate on the balance beam (>60 s) | 3 |
| Attempt balancing on the balance beam and fall off (>40 s) | 4 |
| Attempt balancing on the balance beam and fall off (>20 s) | 5 |
| Fall off and not attempt balancing on the balance beam and fall off (< 20 s) | 6 |
| Reflex loss and abnormal motion | 4 |
| Auricle reflex (shake heads in case of touching external auditory canal) | 1 |
| Corneal reflex (blink in case of slightly touching cornea with silk cotton) | 1 |
| Panic reflex (make motor reaction to the noise of quickly flipping hardboard) | 1 |
| Depressive psychosis, myoclonus and myodystony; | 1 |

18-Score Modified Neurological Severity Score, normal mice= 0, and maximum= 18; the higher the score is, the more severe the nerve injury is, and specific outcome evaluation is shown in table 3:

**Table 3: Outcome evaluation**

| 0 | 1-6 | 7-12 | 13-18 |
|---|---|---|---|
| Normal mice | Mild impairment | Moderate impairment | Severe impairment |

### 2. Experiment results

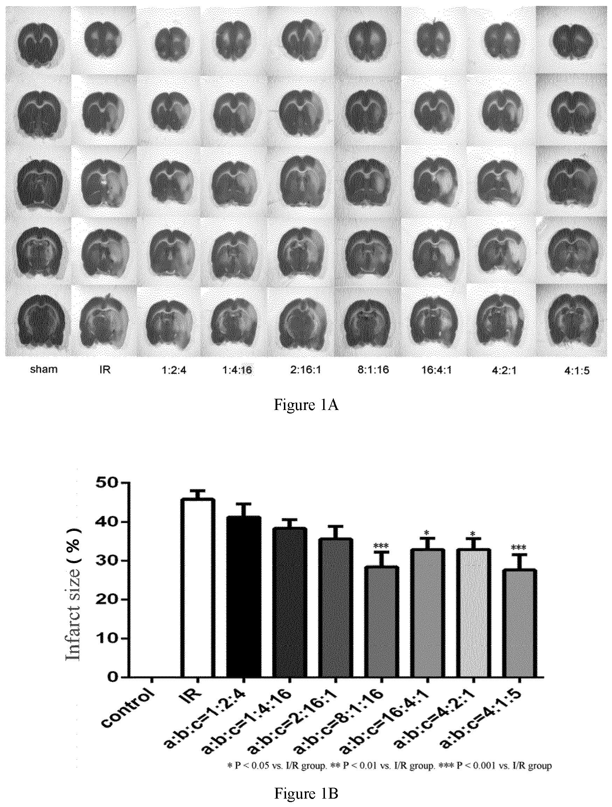

2.1 TTC staining results: as shown in FIGS. 1A and 1B.

FIG.1A shows a TTC staining image of brain tissue slices in each group of rats after perfused for 24h. In the figure, white region denotes infarct region. FIG.1B is a statistical graph showing TTC infarct size in different groups (specific results are shown in table 4).

Compared with sham-operated group, rats in I/R group suffer obvious infarct in right hemisphere. Four compatibility groups (8:1:16, 16:4:1, 16:8:4 and 4:1:5) may reduce the size of cerebral infarction, of which compatibility groups (8:1:16 and 4:1:5) may obviously reduce the size of cerebral infarction (FIGS.1A and 1B).

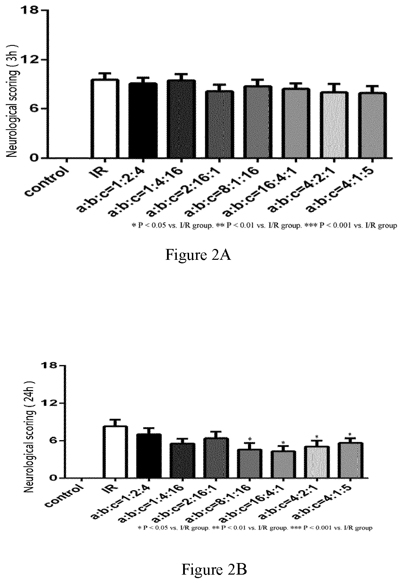

2.2 The neurological scoring results are shown in FIGS. 2A and 2B (respectively showing mNSS results in each group of rats after reperfused for 3h and 24h) and table 4.

**Table 4: Infarct size of different groups of rats, and neurological scoring 3 hours and 24 hours ater reperfusion (mean value ± standard error)**

| Group | Size of cerebral infarction (%) | Neurological scoring 3 hours after reperfusion | Neurological scoring 24 hours after reperfusion |
|---|---|---|---|
| Sham -operated group | 0.0 | 0.0 | 0.0 |
| Ischemia reperfusion group | 43.67± 1.940* | 10.20 ± 0.4667* | 8.800 ± 1.031* |
| Group 1:2:4 | 38.69 ± 3.425* | 9.000 ± 0.4944* | 8.100 ± 0.7667* |
| Group 1:4:16 | 36.74 ± 1.824* | 9.500 ± 0.8596* | 6.000 ± 0.8165* |
| Group 2:16:1 | 35.99 ± 3.069 | 9.100 ± 0.7371* | 6.800 ± 0.7860* |
| Group 8:1:16 | 27.13 ± 3.699*#† | 8.600 ± 0.7630* | 4.600 ± 0.9684*#† |
| Group 16:4:1 | 30.99 ± 2.436*# | 8.500 ± 0.5426* | 4.000 ± 0.6325*#†Δ |
| Group 4:2:1 | 31.06 ± 3.074*# | 8.000 ± 0.9888* | 4.900 ± 0.8492*#† |
| Group 4:1:5 | 24.06 ± 3.621*#†‡Δ | 7.900 ± 0.8492* | 5.600 ± 0.4761 *# |

| | | | |
|---|---|---|---|
| Note: □ denotes a ratio to the sham-operated group, and P < 0.05. # denotes a ratio to the ischemia reperfusion group, and P < 0.05. † denotes a ratio to group 1:2:4, and P < 0.05. ‡ denotes a ratio to group 1:4:16, and P < 0.05; Δ denotes a ratio to group 2:16:1, and P < 0.05. | | | |

### The results of FIGS.2A and 2B and table 4 indicate:

Compared with the sham-operated group (control), the neurological scoring of rats after reperfused for 3 hours and 24 hours significantly decreases; the 7 compatibility groups do not improve the decrease of neurological scoring of rats after reperfused for 3 hours. But for the rats after reperfused for 24 hours, four groups (8:1:16, 16:4:1, 4:2:1 and 4:1:5) may significantly improve the neurological scoring.

By the experiment above, it can be seen that when the ratio of salvianolic acids (a), to *Panax Notoginseng* saponins 9b) and total saponins of *Astragalus* (c) is within the range of (4-16): (1-8): (1-16), preferably, (4-16): (1-4): (1-16), the composition may improve ischemia reperfusion injury well.

In the following experiment, thrombolysis-caused ischemia reperfusion injury is set as an example, and different groups within the range of optimal ratio determined by the above experiment are selected to study the protective effect of the traditional Chinese medicinal composition of the present invention on the thrombolysis-caused ischemia reperfusion injury, thus facilitating thrombolysis. But the above experiment does not limit the protective scope of the present application, since ischemia reperfusion injury caused by any reason shows excessive free radicals, cell calcium overload, inflammatory response, etc., and may be treated by consistent therapeutic methods. The experiment of the present invention verifies that the traditional Chinese medicinal composition of the present invention may treat ischemia reperfusion injury caused by thrombolysis, and accordingly it can be derived that the traditional Chinese medicinal composition of the present invention may also treat ischemia reperfusion injury caused by other reasons.

### Example 2: Thrombolysis promotion to the thrombus of mice carotid artery and protective effect on cerebral ischemia reperfusion injury after thrombolysis

### 1. Animal experimental model building and experimental grouping

### 1.1 Animal model building

21±2 g of male mice C57BL/6 at clean level were purchased from Animal Center of the Peking University Health Science Center. Mice were fed with free access to food and water under the conditions of 12h illumination/darkness alternation at 23 ± 2 □ and 45 ± 5%. Mice were fasted 12 hours before the experiment but free access to drinking water.

Mice were anesthetized via intraperitoneal injection by pentobarbital sodium (2%, 45 mg/kg), and neck kin was sterilized. A median incision was cut on the neck, exposed and isolated to obtain arteria carotis communis; a waterproof cushion was used to isolate arteria carotis communis with the length of about 2.5-3 mm from surrounding tissues. The isolated arteria carotis communis was wrapped by a filter paper soaked by 10% FeCl3 for about 1 mm (width), and the filter paper was removed 3 minutes later, the outside of blood vessel was washed by 0.9% normal saline. The waterproof cushion was removed. The neck incision was sutured. The start of Fe3+ stimulation was defined as the beginning of ischemia, and those mice meeting the following conditions were selected into the group: the blood vessel was blocked at the maximum diameter of the neck thrombus 10 minutes after ischemia; the blood flow of the carotid artery distal end was less than 20% of the base value 15 minutes after ischemia; and the thrombus was stable 4.5 hours after ischemia (before reperfusion); the carotid artery vessel at 60% and more of the diameter was blocked by the maximum diameter of the thrombus.

### 1.2 Experiment grouping

1.2.1 Study on the protective effect of the traditional Chinese medicinal composition of the present invention in different proportions on ischemia reperfusion injury after thrombolysis

Mice were treated by medicaments or normal saline according to random grouping results 4.5 hours after ischemia. Mice femoral vein was intubated, and left femoral vein was injected tPA (tissue plasminogen activator) or normal saline; right femoral vein was injected the traditional Chinese medicinal composition of the present invention or normal saline; 10% of the total dose was injected via an injection pump, and the rest 90% were continuously injected intravenously for 1 hour with the rate of 0.1 ml/h. Three proportion groups were selected to the traditional Chinese medicinal composition of the present invention respectively below:
group T541 (total saponins of *Astragalus:* salvianolic acids: *Panax Notoginseng* saponins=5:4:1);
group T141 (total saponins of *Astragalus:* salvianolic acids: *Panax Notoginseng* saponins=1:4:1)
group T582 (total saponins of *Astragalus:* salvianolic acids: *Panax Notoginseng* saponins=5:8:2).

C57BL/6 mice were divided into 10 groups below according to a random number table:
(1) sham-operated group, Fe3+ stimulation was replaced by normal saline during model building;
(2) high-dose T541 basal group (20 mg/kg T541 was given on the basis of sham-operated group, abbreviated for a basal group);
(3) thrombus group (isovolumetric normal saline was given);
(4) high-dose T541 basal group (20 mg/kg T541 was given separately);
(5) tPA thrombolysis group (tPA was given);
(6) low-dose T541 group (tPA + 5 mg/kg T541 was given);
(7) medium-dose T541 group (tPA + 10 mg/kg T541 was given);
(8) high-dose T541 group (tPA+ 20 mg/kg T541 was given separately);
(9) T141 group (tPA + 12 mg/kg T141 was given);
(10) T582 group (tPA + 15 mg/kg T582 was given).

TPA was given according to a clinical equivalent dose of 10 mg/kg. The mode of administration in each group is shown in table 5.

**Table 5: Animal experimental grouping method and mode of administration**

| | Administration via left femoral vein | Administration via right femoral vein | Total saponins of *Astragalus:* salvianolic acids: *Panax Notoginseng* saponins |
|---|---|---|---|
| Sham -operated group | Normal saline | Normal saline | - |
| High-dose T541 basal group | Normal saline | 20 mg/kg T541 | 5:4:1 |
| Thrombus group | Normal saline | Normal saline | - |
| High-dose T541 thrombolysis group | Normal saline | 20 mg/kg T541 | 5:4:1 |
| tPA thrombolysis group | 10 mg/kg tPA | Normal saline | - |
| Low-dose T541 group | 10 mg/kg tPA | 5 mg/kg T541 | 5:4:1 |
| Medium -dose T541 group | 10 mg/kg tPA | 10 mg/kg T541 | 5:4:1 |
| High-dose T541 group | 10 mg/kg tPA | 20 mg/kg T541 | 5:4:1 |
| T141 group | 10 mg/kg tPA | 12 mg/kg T141 | 1:4:1 |
| T582 group | 10 mg/kg tPA | 15 mg/kg T582 | 5:8:2 |

Left femoral vein was given 10 mg/kg tPA (clinical equivalent dose) or isovolumetric normal saline as a control group. Right femoral vein was respectively given several groups of the traditional Chinese medicinal composition of the present invention, namely, low/medium/high-dose T541 group, T141 group, T582 group or isovolumetric normal saline as a control group.

Low/medium/high-dose T541 group, T141 group or T582 group was administrated with tPA at the same time. Such mode of administration may not only evaluate thrombolysis promotion of the traditional Chinese medicinal composition of the present invention, but also study the therapeutical effect thereof on ischemia reperfusion injury caused by thrombolysis. Observing targets and the quantity of each group of animals in the experiment are shown in table 6.

**Table 6: Observing targets and the quantity of animals in each group the experiment**

| | Surviv al rate (neuro logical scorin g) | Thrombus (infarct size) (cerebral blood flow) | Bleedi ng | Evans Blue leaka ge | Microcircul ation (brain dry/wet weight ratio) | Immunohistoche mistry (immunofluoresc ence) | Electron microsco pe | Meris tem | In total |
|---|---|---|---|---|---|---|---|---|---|
| Sham-ope rated group | 10 | 6 | 6 | 6 | 6 | 3 | 3 | 6 | 46 |
| Basal group | 6 | 6 | 6 | 6 | 6 | 3 | 3 | 6 | 42 |
| Thrombus group | 9 | 6 | 6 | | | | | | 21 |
| High-dos e T541 thrombol ysis group | 8 | 6 | 6 | | | | | | 18 |
| tPA thrombol ysis group | 8 | 6 | 6 | 6 | 6 | 3 | 3 | 6 | 44 |
| Low-dose T541 group | 8 | 6 | 6 | 6 | 6 | 3 | 3 | 6 | 20 |
| Medium-dose T541 group | 8 | 6 | 6 | | | | | | 20 |
| High-dos e T541 group | 9 | 6 | 6 | | | | | | 48 |
| T141 | 6 | 6 | 6 | | | | | | 15 |
| group | | | | | | | | | |
| T582 group | 6 | 6 | 6 | | | | | | 18 |
| In total | 76 | 60 | 60 | 24 | 24 | 12 | 12 | 24 | 292 |

The number of 24 h survival rate and neurological scoring in each group is not less than 6. The number of thrombus observation, triphenyltetrazolium chloride (TTC) infarct size staining, cerebral blood flow and bleeding in each group is 6. The sham-operated group, high-dose T541 basal group, tPA thrombolysis group and optimal drug concentration group were selected for further observation. The number of Evans Blue leakage, dynamic visual observation of microcirculation, dry/wet weight ratio of model lateral ventricle and molecular biological indicators in each group is 6; the number of immunofluorescent staining and electron microscope in each group is 3.

The survival rate, neurological scoring, change of carotid artery ischemia size and change of cerebral blood flow perfusion of mice after reperfused for 24 hours were recorded; and the optimal drug concentration was selected for further analysis.

The dynamic change of microcirculation of mice brain was observed by a dynamic microcirculation observation system of an upright microscope after reperfused for 24 hours; the albumin seepage of middle cerebral artery region, anterior artery region and brain surface postcapillary venule was recorded.

After reperfused for 24 hours, mice were killed to take brain, so as to observe the degree of cerebral hemorrhage and infarct size, measure the hemoglobin content in brain tissues after bleeding, detect dry/wet weight ratio and Evans Blue leakage of brain tissues, reflect the condition of tissue edema, observe the severity order of apoptosis by TUNEL staining brain tissues, observe the change of blood brain barrier of the blood vessel when injured by making Western blotting analysis on connexins Occludin, Claudin-5, JAM-1, ZO-1, VE-cadherin and laminin, namely, the component of basement membrane and by conducting immunofluorescent staining on partial connexins and laminin, detect the change of the structure after cerebral ischemia reperfusion by a scanning electron microscope and TEM, and measure the change on the activity of brain tissues MDA, 8-OHdG, ATP/ADP/AMP and mitochondrial complexes I, II and IV by enzyme-linked immunosorbent assay (ELISA).

1.2.2 Comparison of therapeutical effect on reperfusion injury caused by thrombolysis of arteria carotis communis between the traditional Chinese medicinal composition of the present invention and individual ingredient of the composition

To compare the therapeutical effect on reperfusion injury caused by tPA thrombolysis between the traditional Chinese medicinal composition of the present invention and each ingredient (total saponins of *Astragalus,* the experiment was divided into the following group (tPA is combined with T541, individual ingredient thereof and compatibility of any two ingredients) specifically as follows: tPA+ total saponins of *Astragalus* (tPA+HQ), tPA+ salvianolic acids (tPA+DS), tPA+*Panax Notoginseng* saponins (tPA+SQ), tPA+total saponins of *Astragalus* + salvianolic acids (tPA+HQ+DS), tPA+ total saponins of *Astragalus* + *Panax Notoginseng* saponins (tPA+HQ+SQ), tPA+ salvianolic acids + *Panax Notoginseng* saponins (tPA+DS+SQ) and tPA+ T541. Detailed grouping is shown in table 7. Normal saline served as an isovolumetric control. The usage number in each group is 6.

**Table 7: T541 grouping conditions of separated prescription and drug administration in each group**

| | Administration via left femoral vein | Administration via right femoral vein | Number |
|---|---|---|---|
| Sham-operated group | Normal saline | Normal saline | 6 |
| High-dose T541 basal group | Normal saline | 20 mg/kg T541 | 6 |
| tPA thrombolysis group | 10 mg/kg tPA Normal saline | Normal saline | 6 |
| tPA+HQ thrombolysis group | 10 mg/kg tPA Normal saline | 10 mg/kg total saponins of *Astragalus* | 6 |
| tPA+SQ thrombolysis group | 10 mg/kg tPA Normal saline | 2 mg/kg *Panax Notoginseng* saponins | 6 |
| tPA+DS thrombolysis group | 10 mg/kg tPA Normal saline | 8 mg/kg salvianolic acids | 6 |
| tPA+HQ+SQ thrombolysis group | 10 mg/kg tPA Normal saline | 10 mg/kg total saponins of *Astragalus* and 2 mg/kg *Panax Notoginseng* saponins | 6 |
| tPA+HQ+DS thrombolysis group | 10 mg/kg tPA Normal saline | 10 mg/kg total saponins of *Astragalus* and 2 mg/kg salvianolic acids | 6 |
| tPA+SQ+DS thrombolysis group | 10 mg/kg tPA Normal saline | 2 mg/kg *Panax Notoginseng* saponins and 2 mg/kg salvianolic acids | 6 |
| High-dose T541 group | 10 mg/kg tPA Normal saline | 20 mg/kg T541 | 6 |
| In total | | | 54 |

### 1.3 Cell experimental procedure, grouping and observing targets

### 1.3.1 Cell experimental procedure and grouping

Establishment of rat brain endothelial cell H/R model: rat brain endothelial cells were purchased from ATCC, and then subcultured to 5-6 generations in normal conditions for further experiment.

It was divided into: (1) normal control; (2) model group: rat brain endothelial cells were cultured in hypoxia condition for 4.5 hours and then cultured in reoxygenation condition for 3 hours; 20 mcg/ml of tPA was added at the beginning of reoxygenation; (3) high-dose T541 group: rat brain endothelial cells were cultured in hypoxia condition for 4.5 hours and then cultured in reoxygenation condition for 3 hours; 20 mcg/ml of tPA and 400 mcg/ml of T541 were added at the beginning of reoxygenation; (4) sub-high-dose T541 group: rat brain endothelial cells were cultured in hypoxia condition for 4.5 hours and then cultured in reoxygenation condition for 3 hours; 20 mcg/ml of tPA and 40 mcg/ml of T541 were added at the beginning of reoxygenation; (5) medium-dose T541 group: rat brain endothelial cells were cultured in hypoxia condition for 4.5 hours and then cultured in reoxygenation condition for 3 hours; 20 mcg/ml of tPA and 4 mcg/ml of T541 were added at the beginning of reoxygenation; (6) sub-low-dose T541 group: rat brain endothelial cells were cultured in hypoxia condition for 4.5 hours and then cultured in reoxygenation condition for 3 hours; 20 mcg/ml of tPA and 0.04 mcg/ml of T541 were added at the beginning of reoxygenation.

Cellcounting Kit-8 (CCK-8, MedChem Express, China) was used to test the difference of viability between cells in normal group and cells processed by different medicaments. In the experiment, it was divided into: (1) normal control; (2) high-dose T541 basal group: normal cells were processed by 400 mcg/ml for 3 hours; (3) sub-high-dose T541 basal group: normal cells were processed by 40 mcg/ml for 3 hours; (4) medium-dose basal group: normal cells were processed by 4 mcg/ml for 3 hours; (5) sub-low-lose basal group: normal cells were processed by 0.4 mcg/ml for 3 hours; (6) low-dose basal group: normal cells were processed by 0.04 mcg/ml for 3 hours.

### 1.3.2 Observing targets of cell experiment

Cell viability was tested by Cell-Counting Kit 8 (CCK8) to reflect the influence of the reagents at different concentrations on normal endothelial cells and detect the change of content of connexins Claudin-5, junctional adhesion molecule JAM-1 and VE-cadherin; immunofluorescent staining was conducted to partial connexins and cytoskeleton F-actin; Western blotting analysis was applied to detect the change of content of the apoptosis-related protein Bax (B cell lymphoma 2-related protein X) and Bcl-2 (B cell lymphoma-2); Western blotting analysis was applied to detect the change of content of matrix metalloproteinase (MMP)-3 and its precursor pro-MMP-3.

### 2. Test method

### 2.1 Observation of thrombus in mice neck

Carotid thrombus of mice was dynamically observed by an upright microscope. Anesthetized mice were placed on a board in supine position, a median incision was cut on the neck; arteria carotis communis was exposed and separated; femoral vein was given Acridine red fluorescence labeling of blood platelet to observe the change of carotid thrombus of mice before ischemia (namely, a base value), 10 minutes after ischemia, 4.5 hours after ischemia (namely, before reperfusion), 1 hour after reperfusion, 2 hours after reperfusion and 24 hours after reperfusion. There was no thrombus before ischemia (namely, a base value); the blood vessel was blocked by thrombus at a maximum diameter 10 minutes after ischemia; blood flow at the distal end of carotid artery decreased to 20% of the base value below 15 minutes after ischemia; the thrombus was stable 4.5 hours after ischemia (namely, before reperfusion). The success criterion is that the size of thrombus blocks 60% of the vascular area of carotid artery and above; the success rate of entry is up to 90.9%. Excepting for the sham-operated group and each basal group, the rest groups were randomly allocated after building models successfully.

### 2.2 Measurement of cerebral blood flow

A computer-linked Laser Doppler Flowmetry was used to measure the blood flow of cerebral cortex in the dominant region of bilateral middle cerebral arteries. Anesthetized mice were placed on a board in supine position; skin on the parietal bone of both sides was cut to fully expose the parietal bone; a computer-linked low-energy He-Ne laser probe was placed away 16-18 cm on the parietal bone to detect the blood flow of pia mater in the blood supply region of bilateral middle cerebral arteries of mice before ischemia (namely, a base value), 10 minutes after ischemia, 4.5 hours after ischemia (namely, before reperfusion), 1 hour after reperfusion, 2 hours after reperfusion and 24 hours after reperfusion. A cerebral blood flow analysis software LDPIwin 3.1 (PeriScan PIM3 System, PERIMED, Stockholm, Sweden) was used to calculate the blood flow of cerebral cortex in the same region dominated by bilateral MCA.

### 2.3 Neurological scoring

Neurological scoring was conducted to each group of mice after reperfused for 24 hours based upon scoring standards (15 scores) (Garcia JH et al. Stroke. 1995; 26:627-634.) with slight modification:
Mice capacity of autonomic activity: mice were put into a 25 × 15 cm cage to record their motion trails within 3 minutes; mice incapable of moving basically were denoted as 0; mice with little activity and not touching any wall of the cage were denoted as 1; mice touching any one or more walls were denoted as 2; mice touching three walls and above were denoted as 3;
Mice limbs symmetry experiment: mice tails were lifted to observe the motion trails of their bodies; mice with completely asymmetric bodies were denoted as 0; mice with almost asymmetrical bodies were denoted as 1; mice with moderately asymmetrical bodies were denoted as 2; mice with almost symmetric bodies were denoted as 3;
Measurement of open field test: free motion of mice was observed; mice without any motion were denoted as 0; mice circling around were denoted as 1; mice tilting to one side to make curvilinear motion were denoted as 2; mice making rectilinear motion were denoted as 3;
Beam balance test: 50 × 5 × 2 cm of wooden strips at the position away 10 cm from the ground, then mice were put on one end thereof to observe their motion trails on the balance beam; mice falling off the balance beam were denoted as 0; mice hanging on the balance beam were denoted as 1; mice standing on the balance beam were denoted as 2; mice moving on the balance beam were denoted as 3;
Mice vibrissa touching: vibrissa at both sides of mice was touched by a stick; compared with the right side, mice making no response on left side were denoted as 0; mice making a weak response on left right were denoted as 2; mice making responses at both sides were denoted as 3.

Mice neurological scoring: a sum of the individual score of the above five experiments is calculated; 15 scores denote normal mice and 0 denote dead mice.

Another scoring method is introduced hereafter. Modified neurological severity scores, mNSS, the score is within the range of 0-18. The scoring method is referring to 1.4 of Example 1.

### 2.4 Measurement of cerebral hemorrhage and infarct size

After reperfused for 24 hours, mice were perfused via heart by a PBS buffer precooled at 4□ and separated brain via craniotomy; the brain was cut from front to back into 5 slices in a brain stereotaxic apparatus; bleeding condition was rapidly shoot by a stereoscopic microscope; then slices were stored at -80 □; and hemoglobin was measured by a hemoglobin spectrophotometry cassete.

After reperfused for 24 hours, mice were perfused via heart by a PBS buffer precooled at 4□ and separated brain via craniotomy; the brain was cut from front to back into 5 slices in a brain stereotaxic apparatus; bleeding condition was rapidly shoot in cold condition; then slices were put into 2% triphenyltetrazolium chloride, TTC for incubation for 15 minutes at -80□ ; TTC staining was conducted. Non-infarct region was stained red and infarct region was not stained and still white. Brain TTC staining images were shoot and IamgeJ (Bethesda, MD, USA) was used to calculate a percentage of TTC staining infarct size in the total area of brain, and evaluate the degree of cerebral infarction.

### 2.5 Microcirculation observation, measurement of Evens Blue leakage and brain tissue dry/wet weight ratio

Plasma albumin leakage: femoral venous cannula of mice was conducted under a stereoscopic microscope (PE/08, outer diameter: 0.36 mm and inner diameter: 0.20 mm). Mice were fixed on an observation board for brain microcirculation in prone position; parietal skin of mice was cut off to expose right parietal bone; the whole parietal bone was thinned by grinding by a hand-held cranial drill till there is only one layer of soft bone cortex under the stereoscopic microscope. FITC-labelled plasma albumin (excitation wavelength: 420-490 nm, emission wavelength: 520 nm) was slowly injected via femoral vein. 10 minutes later, fluorescence images of postcapillary venule, artery in the dominant region of middle cerebral artery and artery in the dominant region of anterior cerebral artery among 30-50 mm (diameter) on the surface of the mice brain after reperfused for 24 hours by an upright fluorescent microscope linked to hypersensitive fluorescent CCD; then leakage of plasma albumin was analyzed. Fluorescence intensity inside (Iv) and outside (Ii) venule was calculated by an image analysis software ImageJ (Bethesda, MD, USA); the leakage of plasma albumin is denoted by Ii/Iv.

Evens Blue leakage: after reperfused for 21 hours, mice were slowly injected an Evens Blue dye (2%, 4 ml/kg) dissolving in normal saline via femoral vein. 3 hours later, mice were perfused 15-20 ml of normal saline till auricula dextra flows colourless liquid, then the brain was immediately separated and immobilized in a 3% paraformaldehyde for 3 hours, then taken out, the brain was cut into 5 slices and shoot; left and right brain was separated and respectively weighed. Left and right brain was respectively added to an EP tube containing 1 ml of 50% trichloroacetic acid, homogenated and centrifuged to obtain the supernatant. An Evens Blue standard was prepared and the content of Evens Blue was detected by a multimode reader (exciting light: 620 nm, emitting light: 680 nm). The content of Evens Blue was denoted by the microgram of Evens Blue in per gram of brain tissue.

Brain tissue dry/wet weight ratio: after reperfused for 24 hours, mice were immediately anesthetized; the head was cut off to take brain; left and right brain was respectively weighed (denoted as wet weight). Afterwards, the brain was placed into a 60□ drying oven for drying for 72 hours, and then weighed (denoted as dry weight). Brain tissue dry/wet weight ratio is calculated by (wet weight-dry weight)/wet weight × 100%.

### 2.6 Electron microscopy observation

Transmission electron microscope (TEM): after reperfused for 24 hours, anesthetized mice were perfused 3% glutaraldehyde for 40 minutes via ventriculus sinister at a rate of 3 ml/min; the brain was taken, and the mice right cortex was cut into small pieces (1 mm x 1 mm x 1 mm); small pieces were immobilized in 3% glutaraldehyde for 30 minutes at room temperature or stored over night at 4□. The brain pieces were washed on a table containing sucrose for 3 times for 15 minutes, immobilized by osmic acid for 2 hours at room temperature, washed by 0.2; PBS for 3 times for 15 minutes, dehydrated for 15 minutes respectively by 30%, 50%, 70% and 90% acetone, and dehydrated for 15 minutes for 15 minutes by 100% acetone, then preimpregnated into a pre-embedding agent mixed by 100% acetone and embedding agent according to a ratio of 1:1; and kept over the night at 37□; then brain pieces were impregnated by a pure embedding agent for 24 hours at 37□, and cured for 48 hours, then naturally cooled; afterwards, brain pieces were trimmed and cut into slices with the thickness of 60 nm. The slices were stained by uranyl acetate and lead citrate, and observed and shoot by a TEM (JEM 1230, JEOL, Tokyo, Japan).

Scanning electron microscope (SEM): after reperfused for 24 hours, anesthetized mice were perfused 3% glutaraldehyde for 40 minutes via ventriculus sinister at a rate of 3 ml/min; the brain was taken, and the right cortical penumbra was cut off and immobilized in glutaraldehyde for 2 hours. After washed by a PBS phosphate buffer, mice were immobilized in 1% osmium tetroxide for 2 hours; a proper section was taken and fixed on a metal support. Tissue blocks were plated gold, observed and shoot by an SEM (JSM-5600LV, JEOL, Tokyo, Japan).

### 2.7 Western blotting analysis and enzyme-linked immunosorbent assay

After reperfused for 24 hours, mice were perfused by normal saline; Western blotting analysis was conducted to the penumbra field of infarcted right cerebral cortex. After perfused, mice brain tissue was taken out; cerebellum and prefrontal lobe were cut off; the brain tissue of right brain close to 1 cm of the central joint of left and right hemispheres along the sagittal view; infarcted penumbra field of the rest tissues was cut off along an 45° angle; the cerebral cortex of the penumbra field was peeled off and preserved at low temperature. Right cerebral cortex was taken and added 1 × RIPA lysate and a Cocktail protease inhibitor (1:100, Cell Signaling Technology, US) for full pyrolysis and ultrasonic treatment, and then 17000 g were centrifuged for 30 minutes. Supernatant was taken and added 5 × loading buffer by volume, boiled for 20 minutes in boiling water, and then preserved at -80□. Excessive supernatant was taken out and quantified by a BCA protein quantification liquor (ThermoScientific, US) to measure absorbance value at 560 nm of ELIASA and to calculate the protein concentration according to a standard curve. Same amount of protein in each group was added to each polyacrylamide gel pores, the gel was concentrated at 80 V and separated by 100 V electrophoretic separation; the separated protein strips were transferred onto a PVDF membrane (Millipore, US) for membrane transferring for 120 minutes at 220 mA. The membrane was sealed in skimmed milk for 1 hour at room temperature to remove nonspecific binding sites, 5% skimmed milk powder-diluted primary antibodies β-actin, claudin-5, occludin, ZO-1 (Abcam, US), VE-cadherin, JAM-1, MMP-3 (Santa Cruz Biotechnology, US), caveolin-1 (Cell Signaling Technology, US), bcl-2, bax, and laminin for incubation over the night at 4□. The membrane was washed by TBST for 10 minutes every other day; a 5% skimmed milk powder-diluted second antibody was added for incubation for 1 hour at room temperature; a luminescent agent (Applygen, China) was added for color development and developing in a dark room. The density of electrophoretic strips was analyzed by an image analysis software Quantityone. The size of all protein strips is denoted by a relative value of a ratio to β-actin ot total interest proteins.

ELISA adsorption kit was used to measure the change on the content of MDA, 8-hydroxyl-2'-deoxyguanosine (8-OHdG), adenosine triphosphate (ATP), adenosine diphosphate (ADP) and adenosine monophosphate (AMP) in the penumbra field of brain tissue cortex of the mode, and the change on the activity of mitochondrial complexes I, II, IV and V.

6 samples were used to repeat the independent experiment for at least three times for each group of data.

### 2.8 Immunofluorescent staining

After reperfused for 24 hours, mice were perfused by a PBS buffer precooled at 4□ via heart; the brain was taken by craniotomy and then immobilized for 12 hours, dehydrated by 30% sucrose, embedded by OTC, cut into 10 micrometer (thickness) of frozen slices by a freezing microtome (CM1800, Leica, Bensheim, Germany); after dried in the air, the slices were put into 0.1 mol/L PBS for washing for 5 minutes; antigen repair was conducted to the slices by 0.01 mol/L sodium citrate in a microwave oven under 600 W at 90□, and then slices were naturally cooled for 2 hours at room temperature. Membrane was broken by PBST for 1 hour at constant temperature of 37□ and constant humidity; the slices were washed by a PBS buffer for 5 minutes for three times, and digested by pepsase for 15 minutes, and then washed by PBS buffer for 5 minutes for three times again. The slices were sealed by sheep serum for 30 minutes, and washed by PBS buffer for 5 minutes for three times, and then added a primary antibody vWF (1:100, Millipore, Temecula, CA, USA) + JAM-1/Occludin/Laminin (1:50, Invitrogen, Camarillo, CA, USA), staying over the night at 4□. The slices were taken out and reheated for 1 hours every other day, washed by PBS buffer for 5 minutes for 3 three times, and added a second antibody for 2 hours, then added Hoechst 33342 (1:100, Molecular Probes) to label cell nucleus, then incubated away from light for 10 minutes at room temperature. An anti-fluorescence quenching mounting medium is used for coverslip mounting. It was observed and shoot by a 63-fold objective of confocal laser scanning microscope (TCS SP5, Leica, Mannheim, Germany).

### 2.9 Statistical analysis

All data are denoted by mean ± SEM; statistics was made to the data by a statistical software GraphPad Prism 6.0, One-Way ANOVA or Two-way ANOVA (cerebral blood flow and neurological scoring); the comparison between two values among groups is corrected by Bonferroni; p<0.05 denotes that difference has statistical significance.

### 3. Experiment results

### 3.1 Change of carotid artery thrombus

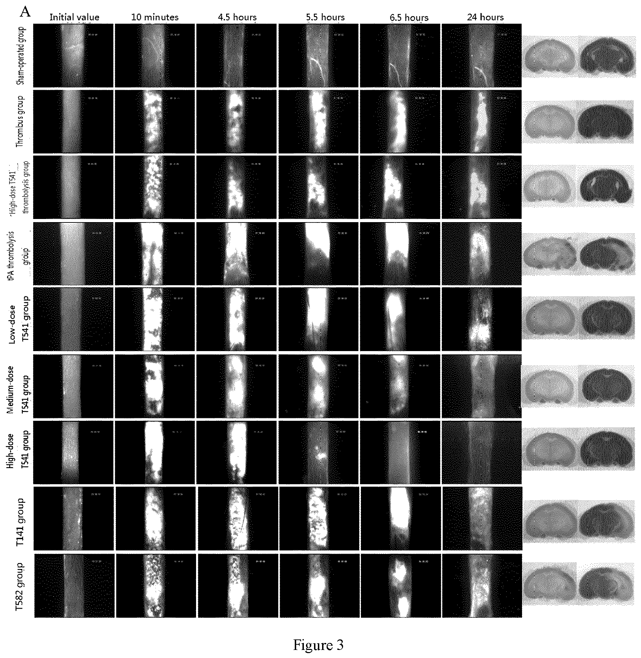

### 3.1.1 Dynamic change of the thrombus of arteria carotis communis in each group of mice: see FIG.3

FIG.3 result displays that compared with sham-operated group, after mice were simulated by FeCl3 filter paper for 10 minutes, thrombosis of arteria carotis communis may be observed in each group. Thrombus is partially dissolved after administrating tPA, but 24 hours later, thrombus in carotid artery may be still observed. T541 dose increases the effect of tPA thrombolysis, especially, the combination of high-dose T541 with tPA may obviously enhance thrombolysis, and thrombus in carotid artery can be significantly dissolved 1 hour after administration.

### 3.1.2 Size of thrombus at each time point: see table 8

**Table 8: Size of thrombus at different time points (N=6)**

| Group | Before FeCl3 simula tion | 10 minutes after FeCl3 simulation | 4.5 hours after FeCl3 simulation | 5.5 hours after FeCl3 simulation (1 hour after administration) | 6.5 hours after FeCl3 simulation (2 hours after administration) | 28.5 hours after FeCl3 simulation (24 hours after administration) |
|---|---|---|---|---|---|---|
| Sham-oper ated group | 0.0 | 0.0 | 0.0 | 0.37±0.37 | 0.28±0.18 | 0.32±0.26 |
| Thrombus group | 0±0 | 25.60±0.89 * | 26.86±1.46* | 23.56±0.78* | 24.35±1.61* | 24.02±1.64* |
| High-dose T541 thrombolys is group | 0±0 | 26.36±0.64 * | 26.17±1.52* | 25.43±1.90* | 25.73±2.27* | 25.3±2.14* |
| tPA thrombolys is group | 0±0 | 25.13±1.34 * | 23.47±1.78* | 22.14±1.36* | 16.64±4.10* | 11.15±2.61*# |
| Low-dose T541 group | 0±0 | 24.86±1.35 * | 22.82±1.16* | 14.06±3.38*# † | 1228±2.57*# † | 7.87±2.74#† |
| Medium-do se T541 group | 0±0 | 25.67±1.79 * | 25.00±1.25* | 15.51±2.19*# † | 9.60±3.24*#† | 3.91±0.43#† |
| High-dose T541 group | 0±0 | 26.88±1.23 * | 25.32±0.56* | 10.80±3.24*# †‡ | 2.78±1.38#†‡ $ | 0.53±0.26#†‡ |
| T141 group | 0±0 | 24.16±0.93 * | 25.15±1.19* | 22.30±1.68* | 18.97±2.73* | 12.57±1.33*# |
| T582 group | 0±0 | 25.56±1.50 * | 22.88±1.19* | 17.71±1.00* | 14.57±1.10*# | 15.19±1.87* |

| | | | | | | |
|---|---|---|---|---|---|---|
| Note: when * is compared with sham-operated group, p<0.05; when # is compared with thrombus group, p<0.05; when † is compared with tPA thrombolysis group, p<0.05; when ‡ is compared with low-dose T541 group, p<0.05; when $ is compared with medium-dose T541 group, p<0.05. | | | | | | |

Results of table 8 show that: compared with thrombus group, the size of thrombus decreases obviously in tPA group 24 hours later. When T541 is simply given, there is no thrombolysis effect. But when T541 is combined with tPA, tPA thrombolysis effect in high/medium/low-dose T541 groups begin to increase at 1 hour; while T141 and T582 groups have no obvious enhancement of thrombolysis effect.

### 3.2 Cerebral blood flow perfusion

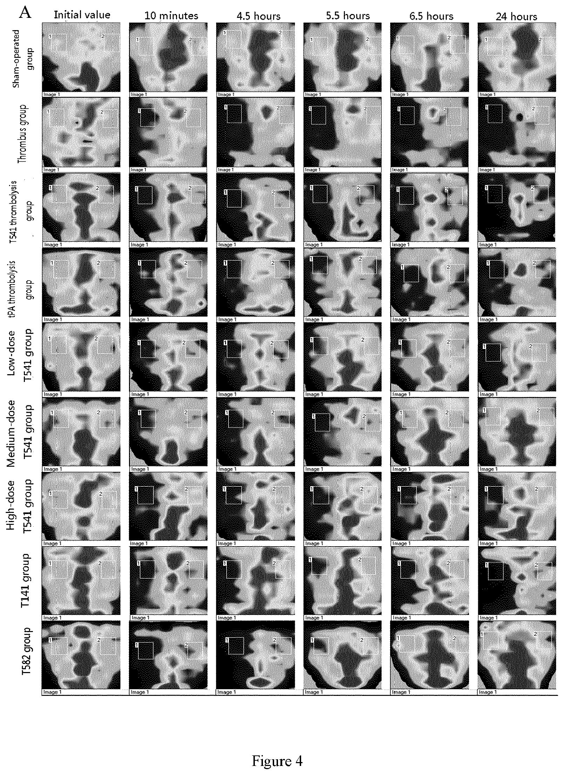

Results of blood perfusion on the brain surface of mice by a Laser Doppler Flowmetry are shown in FIG.4 and table 9

**Table 9: Cerebral blood flow of mice in each group at different time points (N=6)**

| Group | Before FeCl3 simulation | 10 minutes after FeCl3 simulation | 4.5 hours after FeCl3 simulation | 5.5 hours after FeCl3 simulation (1 hour after administrati on) | 6.5 hours after FeCl3 simulation (2 hours after administrati on) | 28.5 hours after FeCl3 simulation (24 hours after administratio n) |
|---|---|---|---|---|---|---|
| Sham-operat ed group | 101.43±1. 08 | 99.65±1.3 7 | 101.33±1. 72 | 99.78±1.16 | 98.93±2.08 | 101.18±1.10 |
| Thrombus group | 100.47±1. 44 | 59.39±3.7 9* | 45.45±1.2 6* | 46.27±1.65* | 41.12±3.13* | 35.80±1.73* |
| High-dose T541 thrombolysi s group | 99.39±1.0 2 | 50.86±5.7 6* | 42.56±4.3 9* | 45.58±428* | 51.72±4.04* | 31.43±2.49* |
| tPA thrombolysi s group | 100.25±1. 19 | 55.20±5.6 9* | 49.58±7.4 4* | 47.56±6.13* | 41.46±4.49* | 42.40±4.61* |
| Low-dose | 9924±1.2 | 56.12±5.2 | 59.12±3.9 | 56.47±6.33* | 57.93±4.11* | 55.51±10.23* |
| T541 group | 0 | 3* | 8* | | | |
| Medium -dos e T541 group | 10025±0. 70 | 49.31±6.3 0* | 51.10±7.2 9* | 51.60±524* | 55.46±7.92* | 68.12±12.05* #† |
| High-dose T541 group | 100.59±1. 70 | 49.34±2.6 1* | 49.83±3.6 5* | 59.63±128* | 75.51±6.18# † | 82.70±1.74#t † |
| T141 group | 99.49±1.6 3 | 56.27±4.1 2* | 42.66±5.9 1* | 54.45±628* | 49.40±8.05* | 39.66±7.84* |
| T582 group | 97.55±3.2 5 | 47.23±4.1 5* | 43.61±2.7 2* | 47.05±1.78* | 5420±6.77* | 40.84±10.21* |

| | | | | | | |
|---|---|---|---|---|---|---|
| Note: when * is compared with sham-operated group, p<0.05; when # is compared with thrombus group, p<0.05; when † is compared with tPA thrombolysis group, p<0.05; when ‡ is compared with low-dose T541 group, p<0.05. | | | | | | |

Results of table 9 show that: there is no difference of brain surface blood flow of mice in each group under initial conditions, and the blood flow of hemispheres is distributed evenly. Excepting for sham-operated group, the blood flow on the surface of lateral brain of mice in each group decreases obviously 10 minutes after building carotid artery thrombus, and 4.5 hours later the brain flood flow does not still get recovery. When high-dose T541 and tPA are simply given, there is no obvious recovery of the brain blood flow. When medium-dose T541 and tPA are combined, the brain blood flow gets recovery obviously 24 hours after administration. When high-dose T541 and tPA are combined, the brain blood flow is obviously higher than the tPA group 2 hours after administration; 24 hours later, the brain blood flow is obviously higher than the combination of high-dose T541 and tPA. But there is no obvious recovery of brain blood flow in the T141 + T582 + tPA group.

### 3.3 Survival rate and neurological scoring

### 3.3.1 Survival rate of mice in each group 24 hours after administration

**Table 10: Change of the survival rate (%) of mice in each group within 24 hours**

| After administ ration/h | Sham-operat ed group | Basal group | Thro mbus group | High-d ose T541 thromb olysis group | tPA throm bolysi s group | Low-d ose T541 group | Mediu m-dos e T541 group | High-d ose T541 group | T141 group | T582 group |
|---|---|---|---|---|---|---|---|---|---|---|
| 0 | 100.0 0 | 100.0 0 | 100.0 0 | 100.0 0 | 100.0 0 | 100.0 0 | 100.0 0 | 100.00 | 100. 00 | 100.0 0 |
| 6 | | | | | 70.00 | 75.00 | | | 66.6 7 | 83.33 |
| 12 | | | | | 60.00 | 62.50 | 87.50 | | 50.0 0 | 50.00 |
| 18 | | | 81.82 | | 50.00 | 50.00 | 62.50 | 90.91 | | |
| 24 | 100.0 0 | 100.0 0 | 81.82 | 90.00 | 50.00 *# | 50.00 *# | 50.00 *# | 81.82* †‡$ | 50.0 0*# | 50.00 *# |

| | | | | | | | | | | |
|---|---|---|---|---|---|---|---|---|---|---|
| Note: when * is compared with sham-operated group, p<0.05; when # is compared with thrombus group, p<0.05; when † is compared with tPA thrombolysis group, p<0.05; when ‡ is compared with low-dose T541 group, p<0.05; when $ is compared with medium-dose T541 group, p<0.05. | | | | | | | | | | |

Results of table 10 show that: there is no dead mouse in sham-operated group and basal group. The survival rate in thrombus group 18 and 24 hours after administration is 81.82%. The survival rate in high-dose T541 thrombolysis group 24 hours after administration is 90%. The survival rate in tPA group 24 hours after administration is 50%; low/medium-dose T541 may not improve the decrease of 24 h survival rate caused by tPA. The survival rate in high-dose T541 group 24 hours after administration is 81.82%; compared with thrombus group, the survival rate increases obviously (81.82% vs 50%). T141 and T582 may not improve the decrease of survival rate caused by tPA either.

### 3.3.2 Neurological scoring 24 hours after administration

18-Score Modified Neurological Severity Score (normal mice= 0; the higher the score is, the more severe the nerve injury is), and a 15-score standard (normal mice=15, dead mice= 0) are applied.

**Table 11: Neurological scoring of mice in each group 24 hours after administration**

| Group | Numb er | 18-Score Modified Neurological Severity Score | 15-score standard |
|---|---|---|---|
| Sham-operated group | 8 | 0.44±0.18 | 14.38±0.50 |
| Basal group | 8 | 0.36±0.15 | 14.33±0.17 |
| Thrombus group | 9 | 9.83±0.70* | 6.2±0.77* |
| High-dose T541 thrombolysis group | 7 | 4.00±1.62# | 7.75±2.36* |
| tPA thrombolysis group | 10 | 7.45±0.73* | 5.82±0.52* |
| Low-dose T541 group | 8 | 1.25±0.16#† | 11.25±0.49#† |
| Medium-dose T541 group | 8 | 2.25±0.41#† | 12.00±0.96#† |
| High-dose T541 group | 9 | 1.27±0.14#† | 13.88±0.44#† ‡ |
| T141 group | 6 | 5.00±2.00*# | 9.00±1.61* |
| T582 group | 6 | 2.33±0.44#‡ | 9.67±1.59* |

| | | | |
|---|---|---|---|
| Note: when * is compared with sham-operated group, p<0.05; when # is compared with thrombus group, p<0.05; when † is compared with tPA thrombolysis group, p<0.05; when ‡ is compared with low-dose T541 group, p<0.05. | | | |

Results of table 11 show that: compared with sham-operated group and basal group, neurological scoring of mice in thrombus group 24 hours after administration is respectively 9.82 (18-Score Modified Neurological Severity Score) and 6.2 (15-score standard). The neurological scoring in high-dose T541 group is 4 (18-Score Modified Neurological Severity Score). TPA may not improve the neurological scoring. Low/medium/high-dose T541 groups obviously improve the neurological scoring, and the effect of high-dose group is obvious superior to low-dose group.

According to the survival rate, neurological scoring, size of neck thrombus and cerebral blood flow perfusion, high-dose T541 group is selected as the optimal drug concentration for further analysis.

### 3.4 Evans Blue leakage, cerebral microcirculation injury and perivascular edema

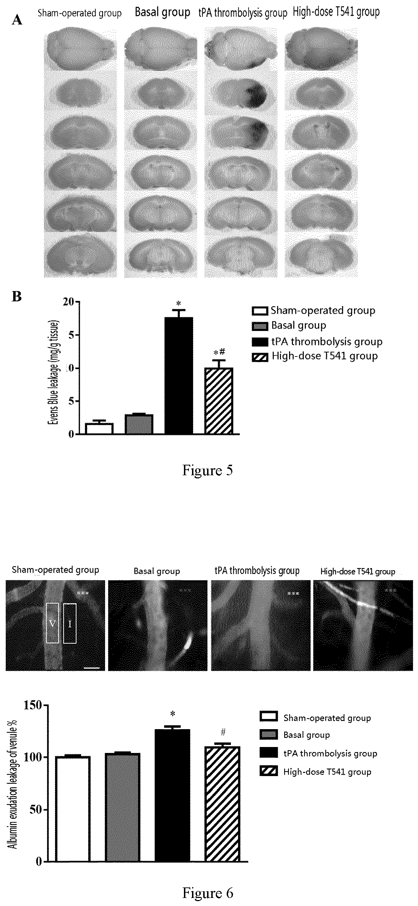

3.4.1 Evans Blue leakage: results of FIG.5 and table 12 show that: mice were given Evens Blue 21 hours after administration for systemic circulation; 3 hours later, Evens Blue in blood circulation was washed out, and then brain was taken and cut into 1 mm (thickness) slices to observe the Evens Blue permeating into brain tissues. Evens Blue obviously seep from brain tissues in tPA thrombolysis group; compared with sham-operated group, the detection result of content is the same; the content of the exudative Evens Blue may be inhibited by 20 mg/kg T541. Evens Blue obviously seep from brain tissues in tPA thrombolysis group; the exudation may be weakened by the combination with high-dose T541.

### 3.4.2 Vascular permeability of brain surface postcapillary venule in ischemic penumbra:

Results of FIG.6 and table 12 show that: 24 hours after thrombolysis, a dynamic visualization method is used to observe the vascular permeability of brain surface postcapillary venule in ischemic penumbra under a living body. The leakage of intravascular albumin is observed within 30 minutes. Plasma albumin obviously seeps in tPA thrombolysis group; high-dose T541 group may significantly inhibit the exudation of plasma albumin.

### 3.4.3 Cerebral perivascular edema, opening number of microvessels, and dry/wet weight ratio 24 hours after administration:

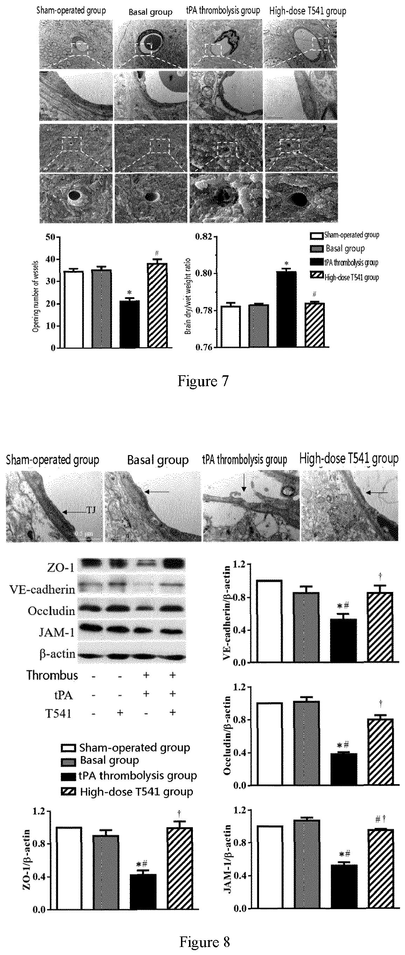

TEM images show that in sham-operated group and high-dose T541 basal group, the vascular structure is complete, and vascular endothelium is smooth and continuous, and closely linked to surrounding tissues. In tPA thrombolysis group, vascular endothelium is rough, and has obvious edema gap with surrounding tissues; edema occurs on the mitochondria of the surrounding tissues. In high-dose T541 group, vascular endothelium is relatively smooth; perivascular edema decreases obviously; the mitochondria structure is compact; and there is no edema gap in the surrounding tissues. (See lines 1 and 2 of FIG.7)

SEM shows that: in sham-operated group and high-dose T541 basal group, the integral structure of cerebral cortex is smooth; 4.5 hours after thrombosis, tPA is administrated; 24 hours later, the integral structure of cerebral cortex is disordered and has an uneven surface; the combination with T541 may obviously improve the status of the integral tissues. 5 visual fields are randomly selected to calculate the opening number of blood vessels of animal's cerebral cortex in each group; thus, it can be seen that compared with sham-operated group and high-dose T541 basal group, the number of blood vessels of brain tissues decreases significantly. 5 500-fold visual fields were randomly selected for each mouse under SEM to count the opening number of cerebral cortex microvessels in right hemisphere penumbra. N=3. (See lines 3 and 4 of FIG.7)

**Table 12: Evans Blue content, albumin exudation ratio and dry/wet weight ratio of brain tissues**

| Group (mean ± standard error) | Evens Blue leakage (N= 6) | Albumin exudation ratio of venule % (N=6) | Opening number of microvessels (N= 3) | Brain dry/wet weight ratio (N= 6) |
|---|---|---|---|---|
| Sham -operated group | 1.56±0.51 | 101.60±1.67 | 34.38±1.32 | 0.780±0.003 |
| Basal group | 2.85±0.23 | 100.60±1.77 | 35.00±1.69 | 0.783±0.001 |
| tPA thrombolysis group | 17.55±1.23* | 125.10±3.53* | 21.07±1.54* | 0.801±0.002* |
| High-dose T541 group | 9.94±1.24# | 110.80±3.38# | 37.80±2.06# | 0.784±0.001# |

| | | | | |
|---|---|---|---|---|
| Note: when * is compared with sham-operated group, p<0.05; when # is compared with tPA thrombolysis group, p<0.05. N≤6. | | | | |

Bar graphs of table 12 and FIG.7 show: statistical results of Evens Blue content, plasma albumin exudation and dry/wet weight ratio 24 hours after administration. TPA may lead to the increase of Evens Blue leakage and plasma albumin, the decrease of opening number of brain microvessels, and the increase of dry/wet weight ratio. T541 may inhibit the above changes caused by tPA. The result shows that tPA thrombolysis may lead to cerebral microvessel exudation and encephaledema, while T541 may inhibit tPA-induced cerebral microvessel exudation and encephaledema.

### 3.5 Change of connexins of cerebral microvascular endothelial cells 24 hours after administration

T541 may improve changes of connexins of cerebral microvascular endothelial cells in cerebral cortex of penumbra field 24 hours after administration. TEM shows that in tPA thrombolysis group, the close adhesion structure of cerebral cortex vessels in penumbra field is opened; the combination of T541 with tPA may shut down the opened endothelial cell adhesion structure, thus recovering the normal connection of cells. (See FIG.8).

Western blotting serves to detect the change of connexins in right cerebral cortex penumbra (see FIG.8 and table 13). It can be seen that tight-junction protein ZO-1, occludin, JAM and adherent connexin VE-cadherin significantly decrease which may be reversed by the combination of T541 with tPA.

**Table 13: Quantitative statistics on connexins of brain tissues 24 hours after administration**

| Group (mean ± standard error) | Number | ZO-1/β-act in | VE-cadherin/β -actin | Occludin/β-actin | JAM-1/β-ac tin |
|---|---|---|---|---|---|
| Sham-operated group | 6 | 1.00±0 | 1.00±0 | 1.00±0 | 1.00±0 |
| Basal group | 6 | 0.90±0.07 | 0.85±0.08 | 1.02±0.06 | 1.07±0.04 |
| tPA thrombolysis group | 7 | 0.42±0.05 *# | 0.53±0.07*# | 0.38±0.02*# | 0.52±0.04* # |
| High-dose T541 group | 7 | 0.99±0.08 † | 0.85±0.09† | 0.80±0.05† | 0.95±0.01# † |

| | | | | | |
|---|---|---|---|---|---|
| Note: when * is compared with sham-operated group, p<0.05; when # is compared with basal group, p<0.05; when † is compared with tPA, p<0.05. | | | | | |

Results of FIG.8 and table 13 hint that: 24 hours after giving tPA thrombolysis at 4.5 hours after thrombosis, the tight junction to cerebrovascular endothelium and adherent junction to basement membrane decline dramatically, and vascular structure is incomplete, which may result in the increase of subsequent vascular permeability and vascular exosmose, thus increasing potential risk of bleeding. T541 may inhibit the connexin injury of cerebrovascular endothelial cell caused by tPA.

### 3.6 Change of the connexin of endothelial cells cultured in vitro after undergoing hypoxia/reoxygenation: see FIG.9 and table 14

To study the influences of tPA and T541 on blood brain barriers, in particular to vascular endothelial cells and basement membrane, rat cerebral microvascular endothelial cells were cultured in vitro; processed under hypoxia conditions for 4.5 hours and reoxygenation conditions for 3 hours to simulate the state of reperfusion after suffering ischemia and thrombolysis in vivo; then cells were cultured under reoxygenation conditions, administrated tPA and T541 for treatment.

A CCK8 kit is used to test the change of activity of normal cells and the normal cells after administrated T541 at different concentrations. Test results show that: the activity of endothelial cells is inhibited in high-dose T541 basal group, there is no significant difference to the activity of endothelial cells in other T541 groups relative to the normal control.

After suffering hypoxia/reoxygenation, brain endothelial cells cultured in vitro are treated by tPA to cause the decline of connexins VE-cadherin, Claudin-5 and JAM-1; T541 (400, 40, 4, 0.4, 0.04 (µg/ml) may recover the expression quantity of connexins in different degrees. 40 µg/ml T541 may recover the quantity of VE-cadherin, Claudin-5 and JAM-1. The combination of T541 with tPA may decrease the content of connexins, which contributes to maintaining the normal cellular morphology of endothelial cells.

T541 may improve the connexin injury of endothelial cells cultured in vitro after suffering hypoxia/reoxygenation

**Table 14: Quantitative statistics on the connexin of endothelial cells cultured in vitro after suffering hypoxia/reoxygenation**

| Group (mean ± standard error) | VE-cadherin/β-actin (N=4) | Claudin-5/β-actin (N=6) | JAM-1/β-actin (N=6) |
|---|---|---|---|
| Normal control | 1.00±0 | 1.00±0 | 1.00±0 |
| Model group | 0.58±0.07* | 0.40±0.06* | 0.64±0.05* |
| High-dose T541 group | 1.05±0.08# | 0.87±0.21 | 0.91±0.04 |
| Sub-high-dose T541 group | 1.04±0.08# | 1.06±0.11# | 1.14±0.11# |
| Medium-dose T541 group | 0.79±0.05 | 0.98±0.12# | 1.03±0.07# |
| Sub-low-dose T541 group | 0.75±0.12 | 0.78±0.17 | 0.97±0.05# |
| Low-dose T541 group | 0.66±0.06* | 0.57±0.09 | 0.90±0.09 |

| | | | |
|---|---|---|---|
| Note: when * is compared with normal control, p<0.05 vs; when # is compared with model group, p<0.05. N≥4. | | | |

Western blotting shows that (FIG.9 and table 14): cerebral microvascular endothelial cells were cultured in vitro, after undergoing hypoxia/reoxygenation and tPA-induced injury, the expression quantity of tight-junction proteins Claudin-5, JAM-1 and adherent connexin VE-cadherin declines, which is consistent with the change of the content of connexins of cerebral cortex tissues cultured in vivo. The decline of expression quantity may be improved by the combination of T541 with tPA according to concentration dependency of the reagents used. 40 mcg/ml T541 is useful to the three kinds of connexins during cell culture. The above results show that T541 may improve the tight-junction and adherent connexins of cerebrovascular endothelial cells, thus decreasing tPA damage on brain microvessels, and lowering the risk of exudation and edema.

### 3.7 Change of major proteins of cerebral hemorrhage and basement membrane

### 3.7.1 Change of hemoglobin and infarct size in brain tissues: see FIG. 10 and table 15

**Table 15: Hemoglobin and infarct size in brain tissues**

| Group (mean ± standard error) | Number | Hemoglobin | Infarct size |
|---|---|---|---|
| Sham-operated group | 6 | 2.83±0.21 | 0±0 |
| Basal group | 6 | 4.50±0.52 | 0±0 |
| tPA thrombolysis group | 7 | 13.55±3.72*# | 15.68±4.81*# |
| High-dose T541 group | 6 | 5.33±1.01† | 5.04±0.68† |

| | | | |
|---|---|---|---|
| Note: when * is compared with sham-operated group, p<0.05; when # is compared with basal group, p<0.05; when † is compared with tPA thrombolysis group, p<0.05. N≥6. | | | |

Mice brain tissues in each group were cut into 1 mm (thickness) slices. Hemorrhage and infarct are observed in the substantial region and lateral cortex of tPA thrombolysis model groups; the phenomena may be eased by the combination of T541 with tPA (see FIG. 10). Right hemisphere tissues of mice in each group were taken to detect hemorrhage; it can be seen that the hemoglobin of tPA thrombolysis group increases obviously, the elevated hemoglobin declines in high-dose T541 group to the level of sham-operated group and basal group (see FIG. 10 and table 15).

### 3.7.2 Change of basement membrane of cerebral ischaemic cortex 24 hours after administration: see FIG.11 and table 16

**Table 16: Statistics on major protein expression of basement membrane in brain tissues 24 hours after administration**

| Group (mean ± standard error) | Number | Collagen IV content | Laminin content |
|---|---|---|---|
| Sham-operated group | 6 | 1.00±0 | 1.00±0 |
| Basal group | 6 | 1.00±0.03 | 0.92±0.11 |
| tPA thrombolysis group | 6 | 0.68±0.04*# | 0.47±0.06*# |
| High-dose T541 group | 6 | 0.90±0.03† | 0.96±0.14† |

| | | | |
|---|---|---|---|
| Note: when * is compared with sham-operated group, p<0.05; when # is compared with basal group, p<0.05; when † is compared with tPA thrombolysis group, p<0.05. | | | |

Results of FIG.11 and table 16 show that: Western blotting serves to detect the change of Collagen IV and Laminin, namely, major components of the basement membrane in right cerebral cortex penumbra; it can be seen that the expression quantity of Collagen IV and Laminin declines sharply in tPA thrombolysis group; and the decline may be reversed by the combination of T541 with tPA (see FIG.11). Results hint that: after mice were administrated tPA for thrombolysis 4.5 hours after ischemia, tight junction and adherent junction of cerebral blood vessel may decline, moreover, the expression quantity of Collagen IV and Laminin decreases, which influences the normal structure of endothelial cells and basement membrane, breaks blood brain barrier and is an important reason to cause tPA-induced hemorrhage risk. The combination of tPA with T541 may maintain the expression of Collagen IV and Laminin, and lower the hemorrhage risk after thrombolysis.

### 3.8 Energy change of brain tissues 24 hours after administration: see FIG.12 and table 17

**Table 17: ATP, ADP, and AMP content in brain tissues 24 hours after administration**

| Group (mean ± standard error) | Number | ATP | ADP | AMP | ADP/A TP | AMP/ ATP |
|---|---|---|---|---|---|---|
| Sham-operated group | 6 | 1.84±0. 08 | 1.81±0. 18 | 1.64±0. 20 | 0.99±0. 12 | 0.88±0. 07 |
| tPA thrombolysis group | 6 | 1.05±0. 13* | 2.51±0. 24* | 2.66±0. 32* | 2.45±0. 10* | 2.68±0. 34* |
| High-dose T541 group | 7 | 1.80±0. 07† | 1.93±0. 13† | 1.86±0. 21† | 1.07±0. 06† | 1.03±0. 11† |
| tPA + *Astragalus* group | 6 | 1.22±0. 13* | 2.02±0. 19† | 1.92±0. 22 | 1.72±0. 21 | 1.64±0. 23† |
| tPA + *Salvia miltiorrhiza* group | 6 | 1.05±0. 08 | 2.54±0. 32 | 2.26±0. 17 | 2.46±0. 27* | 2.21±0. 20† |
| tPA + *Panax Notoginseng* group | 6 | 1.42±0. 21 | 2.45±0. 38 | 2.04±0. 17 | 1.88±0. 35 | 1.52±0. 15† |
| tPA + *Astragalus* + *Salvia miltiorrhiza* group | 6 | 1.44±0. 16 | 2.78±0. 31 | 2.03±0. 16 | 2.01±0. 25* | 1.48±0. 16† |
| tPA + *Astragalus* + *Panax Notoginseng* group | 6 | 1.55±0. 23 | 2.15±0. 12† | 1.99±0. 18† | 1.77±0. 25 | 1.67±0. 30† |
| tPA + *Salvia miltiorrhiza + Panax Notoginseng* group | 6 | 1.44±0. 10 | 2.68±0. 24 | 2.05±0. 24 | 1.88±0. 16 | 1.45±0. 19† |

| | | | | | | |
|---|---|---|---|---|---|---|
| Note: when * is compared with sham-operated group, p<0.05; when † is compared with tPA thrombolysis group, p<0.05. | | | | | | |

Major components of T541, total saponins of *Astragalus,* salvianolic acids and *Panax Notoginseng* saponins are divided into groups by an individual ingredient and combinations in pairs, then compared with T541. Enzyme-linked immunosorbent assay serves to detect the change of ATP, ADP and AMP of cerebral cortex in ischemic penumbra 24 hours after administration; a ratio of ADP/ATP and AMP/ATP is calculated. In tPA thrombolysis group, ATP content of brain tissues decreases significantly; the content of ADP and AMP increases 24 hours after administration; the combination of T541 with tPA may recover the declined ATP and decreases the increased ADP and AMP. In tPA thrombolysis group, the ratio of ADP/ATP and AMP/ATP increases; the former is reversed by the combination of T541 with tPA; the latter gets recovery in tPA + *Astragalus* group, tPA + *Panax Notoginseng* group, in-pair administration team and high-dose tPA + T541 group.

### 3.9 Oxidative stress injury and apoptosis of brain tissues 24 hours after administration

### 3.9.1 MDA and 8-OHdG content, mitochondrial complex activity of brain tissues 24 hours after administration

T541 may improve oxidative stress injury of brain tissues 24 hours after administration to ischemia. Major components of T541, total saponins of *Astragalus,* salvianolic acids and *Panax Notoginseng* saponins are divided into groups by an individual ingredient and combinations in pairs, then compared with T541. Enzyme-linked immunosorbent assay serves to detect the change of ATP, ADP and AMP of cerebral cortex in ischemic penumbra 24 hours after administration; and activity of mitochondrial complexes I, II and IV.

**Table 18: MDA and 8-OHdG content, mitochondrial complex activity of brain tissues 24 hours after administration**

| Group (mean ± standard error) | Nu mbe r | MDA | 8-OH dG | Complex I activity | Complex II activity | Complex IV activity |
|---|---|---|---|---|---|---|
| Sham-operated group | 6 | 19.43± 3.49 | 1.14± 0.11 | 3.19±0.05 | 0.055±0.00 6 | 0.484±0.02 0 |
| tPA thrombolysis group | 6 | 30.08± 2.90* | 1.84± 0.11* | 1.75±0.14 * | 0.025±0.00 5* | 0.267±0.01 6* |
| High-dose T541 group | 6 | 18.84± 1.76† | 1.18± 0.08f | 2.75±0.16 † | 0.051±0.00 4† | 0.464±0.06 2† |
| tPA + *Astragalus* group | 6 | 28.49± 1.70 | 1.38± 0.24 | 1.50±0.15 * | 0.037±0.00 3 | 0.287±0.01 4* |
| tPA + *Salvia miltiorrhiza* group | 6 | 27.78± 1.25 | 1.16± 0.10† | 1.42±0.05 * | 0.035±0.00 3 | 0.279±0.02 5* |
| tPA + *Panax Notoginseng* group | 6 | 25.88± 0.89 | 1.40± 0.14 | 1.39±0.15 * | 0.031±0.00 1* | 0.242±0.00 4* |
| tPA + *Astragalus* + *Salvia miltiorrhiza* group | 6 | 27.48± 1.44 | 1.12± 0.14† | 2.18±0.13 * | 0.043±0.00 7 | 0.326±0.05 5* |
| tPA + *Astragalus* + *Panax Notoginseng* group | 6 | 23.52± 0.52 | 1.40± 0.12 | 2.02±0.14 * | 0.030±0.00 6* | 0.235±0.02 3* |
| tPA + *Salvia miltiorrhiza + Panax Notoginseng* group | 6 | 29.09± 1.86 | 1.38± 0.17 | 2.55±0.12 *† | 0.041±0.00 5 | 0.279±0.00 7* |

| | | | | | | |
|---|---|---|---|---|---|---|
| Note: when * is compared with sham-operated group, p<0.05; when † is compared with tPA thrombolysis group, p<0.05. | | | | | | |

Results of FIG. 13 (oxidative stress injury of brain tissues 24 hours after administration) and table 18 show that: high-dose T541 significantly inhibit the increase of MDA in the penumbra field of cerebral cortex in tPA thrombolysis group; the efficacy of groups tPA + *Astragalus,* tPA + *Salvia miltiorrhiza,* tPA + *Panax Notoginseng* or compatibility groups tPA + *Astragalus* + *Salvia miltiorrhiza,* tPA + *Astragalus* + *Panax Notoginseng,* and tPA + *Salvia miltiorrhiza* + *Panax Notoginseng* is inferior to the efficacy of T541 combined with three ingredients. Similarly, high-dose T541 may significantly inhibit the increase of 8-hydroxyl deoxyguanylic acid (8-OHdG) in the cortex of cerebral ischemic penumbra. Excepting for tPA+ *Astragalus* + *Panax Notoginseng* group, the rest groups of tPA + any one of *Astragalus, Salvia miltiorrhiza* and *Panax Notoginseng* or tPA + *Astragalus* + *Salvia miltiorrhiza* and tPA + *Salvia miltiorrhiza* + *Panax Notoginseng* may not achieve significant efficacy.

During activity detection of mitochondrial complex, the activity of mitochondrial complexes I, II, and IV 24 hours after tPA thrombolysis decreases. TPA + *Salvia miltiorrhiza* + *Panax Notoginseng* group may significantly recover the declined activity of mitochondrial complex I after thrombolysis; tPA + *Astragalus* + *Panax Notoginseng* may recover the activity of mitochondrial complex I; high-dose T541 may recover the activity of mitochondrial complexes I, II and IV. Moreover, high-dose T541 group may further recover the activity of mitochondrial complexes I, II and IV to the level of sham-operated group, which may not be achieved by individual ingredient and in-pair compatibility group.

The experimental result hints that different components of T541 play different roles, in particular to the improvement of mitochondrial complex activity. More interestingly, the single use of total saponins of *Astragalus,* salvianolic acids and *Panax Notoginseng* saponins has no significant difference. But after formulated into T541 group, its combination may lower the increase of MDA in brain tissues, and enhance the activity of mitochondrial complexes I and IV. It shows that the compatibility of ingredients brings synergistic effect instead of a simple additive result.

### 3.9.2 Change of ATP5D content in brain tissues 24 hours after administration: see FIG. 14 and table 19

**Table 19: Content of ATP5D in brain tissues 24 hours after administration**

| Group (mean ± standard error) | Number | ATP5D |
|---|---|---|
| Sham-operated group | 6 | 1.00±0 |
| Basal group | 6 | 1.04±0.07 |
| tPA thrombolysis group | 6 | 0.71±0.05*# |
| High-dose T541 group | 6 | 0.93±0.05† |

| | | |
|---|---|---|
| Note: when * is compared with sham-operated group, p<0.05; when # is compared with basal group, p<0.05; when † is compared with tPA thrombolysis group, p<0.05. N= 6. | | |

Results of FIG.14 and table 19 show that: T541 may recover the declined expression quantity of ATP5D in brain tissues 24 hours after administration.

To sum up, when mice suffer right carotid arterial thrombosis for 4.5 hours, and administrated tPA for thrombolysis for 24 hours, the activity of mitochondrial complexes I, II and IV in cortex tissues of mice ischemic penumbra declines, the expression quantity of ATP5D decreases as well. Cells are lack of energy, resulting in the loss of F-actin structure of cytoskeleton and disordered arrangement, thus leading to the rupture and degradation of tight-junction proteins among endothelial cells and adherent connexins among basement membrane to cause perivascular exudation and edema. T541 may not only inhibit the activity decline of mitochondrial complexes I/II/IV, but also reverses the low expression of ATP5D, thus accelerating the recovery of mitochondrial complexes and improving oxidative stress injury. The recovery of mitochondrial function further lowers the increase of ADP/ATP and AMP/ATP 24 hours after tPA administration, improves the energy supply at the tail end of blood vessel, and maintains normal blood brain barrier.

### 3.9.3 Brain tissue apoptosis staining 24 hours after administration: see FIG. 15 and table 20

**Table 20: Statistics on TUNEL positive cells in brain tissues 24 hours after administration**

| Group (mean ± standard error) | Number | TUNEL positive cell counting |
|---|---|---|
| Sham-operated group | 6 | 0.14±0.10 |
| Basal group | 6 | 0.83±0.41 |
| tPA thrombolysis group | 6 | 27.14±1.77*# |
| High-dose T541 group | 7 | 8.29±2.50† |

| | | |
|---|---|---|
| Note: when * is compared with sham-operated group, p<0.05; when # is compared with basal group, p<0.05; when † is compared with tPA thrombolysis group, p<0.05. N=6 | | |

Results of FIG. 15 and table 20 show that: after mice were administrated for thrombolysis, there are a large number of TUNEL positive cells in lateral cerebral cortex of mice obviously in tPA thrombolysis group. After combined with T541, the population of TUNEL positive cells significantly drops, which may mean that T541 may lower the number of apoptotic cells after receiving single tPA thrombolysis (FIG. 15). There are similar results under TEM; it can be seen from the figure that in tPA thrombolysis group, bodies of neuron shrink and deform; the structure of organelle in cytoplasm is abnormal and density increases significantly; karyotheca is uneven; heterochromatin occurs in cell nucleus. After administrated T541, density of the bodies of neuron basically gets normal, the structure of mitochondria and other organelle returns to normal, and karyotheca is smooth.

### 3.9.4 Brain tissue apoptosis 24 hours after administration: FIG.16 and table 21

**Table 21: Quantification of apoptosis proteins Bax and Bcl-2 in brain tissues**

| Group (mean ± standard error) | Number | Bcl-2 | Bax | Bax/Bcl-2 |
|---|---|---|---|---|
| Sham-operated group | 6 | 1.00±0 | 1.00±0 | 1.00±0 |
| tPA thrombolysis group | 6 | 1.51±0.14 | 5.52±0.78* | 3.82±0.57* |
| High-dose T541 group | 6 | 1.41±0.12 | 1.43±020# | 1.14±0.24# |
| tPA + *Astragalus* group | 6 | 1.47±0.14 | 3.17±0.35*# | 2.32±0.39 |
| tPA + *Salvia miltiorrhiza* group | 6 | 1.35±0.17 | 3.29±0.48*# | 2.65±0.48 |
| tPA + *Panax Notoginseng* group | 6 | 1.17±0.20 | 4.10±0.93* | 4.09±1.34* |
| tPA + *Astragalus* + *Salvia miltiorrhiza* group | 6 | 1.14±0.15 | 1.78±0.08# | 1.79±0.37 |
| tPA + *Astragalus* + *Panax Notoginseng* group | 6 | 1.12±0.03 | 2.68±024# | 2.37±0.16 |
| tPA + + *Panax Notoginseng* group | 6 | 1.43±0.10 | 2.01±0.10# | 1.43±0.12 |

| | | | | |
|---|---|---|---|---|
| Note: when * is compared with sham-operated group, p<0.05; when # is compared with tPA thrombolysis group, p<0.05. N=6. | | | | |

Results of FIG.16 and table 21 show that: T541 may improve apoptosis of brain tissues 24 hours after administration. Major components of T541, total saponins of *Astragalus,* salvianolic acids and *Panax Notoginseng* saponins are divided into groups by an individual ingredient and combinations in pairs, then compared with T541. Western blotting serves to detect the change of content of apoptosis-associated proteins Bax and Bcl-2 in the cerebral cortex of ischemic penumbra 24 hours after administration, and the ratio thereof. After mice were administrated tPA for thrombolysis, the expression of Bax extracted from brain tissues significantly increases. The high expression of Bax may be inhibited by individual total saponins of *Astragalus,* salvianolic acids, *Panax Notoginseng* saponins, any two or three compatibility thereof.

### 3.9.5 Apoptosis of endothelial cells cultured in vitro after undergoing hypoxia/reoxygenation: see FIG.17 and table 22

**Table 22: Quantification of apoptosis proteins Bax and Bcl-2 of endothelial cells cultured in vitro**

| Group (mean ± standard error) | Number | Bcl-2 | Bax | Bax/Bcl-2 |
|---|---|---|---|---|
| Normal control | 6 | 1.00±0 | 1.00±0 | 1.00±0 |
| Model group | 6 | 0.69±0.12 | 2.44±0.51 | 4.57±0.75* |
| High-dose T541 group | 6 | 0.79±0.10 | 1.92±0.27 | 2.60±0.25 |
| Sub-high-dose T541 group | 6 | 0.88±0.10 | 2.01±0.51 | 1.78±0.18# |
| Medium-dose T541 group | 6 | 0.99±0.16 | 2.33±0.47 | 2.31±0.45 |
| Sub-low-dose T541 group | 6 | 0.83±0.11 | 2.72±0.67 | 3.41±0.83* |
| Low-dose T541 group | 6 | 0.56±0.11 | 2.81±0.48 | 4.55±0.47* |

| | | | | |
|---|---|---|---|---|
| Note: when * is compared with sham-operated group, p<0.05; when # is compared with basal group, p<0.05; when † is compared with tPA thrombolysis group, p<0.05. N = 6. | | | | |

Results of FIG. 17 and table 22 show that: T541 may improve the degree of apoptosis of endothelial cells cultured in vitro after undergoing hypoxia/reoxygenation. Western blotting serves to detect the change of expression quantity of Bcl-2 and Bax of cerebral microvascular endothelial cells cultured in vitro and ratio thereof.

It can be seen both in the experiment of in vivo carotid artery thrombolysis and in vitro H/R that after mice were administrated tPA 4.5 hours after ischemia and hypoxia, the ratio of Bax/Bcl-2 associated with brain endothelial apoptosis remarkably increases, and apoptosis increases. The result is consistent with that of TUNEL staining and electron microscope. T541 combined administration may ease the apoptosis caused by tPA. The effect may be related to T541 which promotes the recovery of energy metabolism of mitochondria, thus protecting the expression of connexins of vascular endothelial cells and major proteins of basement membrane.

## Claims

1. A traditional Chinese medicinal composition for preventing and/or treating ischemia reperfusion injury, **characterized in that** the traditional Chinese medicinal composition consists of salvianolic acids, *Panax Notoginseng* saponins and total saponins of *Astragalus;* the weight ratio thereof is (4-16): (1-4): (1-16).

2. The traditional Chinese medicinal composition according to claim 1, **characterized in that** in the traditional Chinese medicinal composition, the weight ratio of salvianolic acids to *Panax Notoginseng* saponins and total saponins of *Astragalus* is (4-16): (1-8): (1-16).

3. The traditional Chinese medicinal composition according to claim 2, **characterized in that** in the traditional Chinese medicinal composition, the weight ratio of salvianolic acids to *Panax Notoginseng* saponins and total saponins of *Astragalus* is (4-16): (1-4): (1-16).

4. The traditional Chinese medicinal composition according to claim 3, **characterized in that** in the traditional Chinese medicinal composition, the weight ratio of salvianolic acids to *Panax Notoginseng* saponins and total saponins of *Astragalus* is (4-8): (1-4): (1-16), or (8-16): (1-4): (1-16).

5. The traditional Chinese medicinal composition according to claim 4, **characterized in that** in the traditional Chinese medicinal composition, the weight ratio of salvianolic acids to Panax Notoginseng Saponins and total saponins of *Astragalus* is (4-8): 1: (5-16), or (4-8): (1-2): (1-5).

6. The traditional Chinese medicinal composition according to claim 5, **characterized in that** the traditional Chinese medicinal composition consists of salvianolic acids, *Panax Notoginseng* saponins and total saponins of *Astragalus* in a weight ratio of 4:1:5.

7. A formulation comprising the traditional Chinese medicinal composition according to any one of claims 1-6, **characterized in that** the formulation consists of the traditional Chinese medicinal composition and a pharmaceutically acceptable carrier.

8. Application of the traditional Chinese medicinal composition according to any one of claims 1-6 in the preparation of a medicament for treating and/or preventing ischemia reperfusion injury.

9. The application according to claim 8, **characterized in that** the ischemia reperfusion injury includes but not limited to, cerebral ischemia reperfusion injury, myocardial ischemia reperfusion injury, renal ischemia reperfusion injury, lower limb ischemia reperfusion injury, ischemia reperfusion injury of spinal cord, retina ischemia reperfusion injury, flap ischemia reperfusion injury, or ischemia reperfusion caused by the following reasons: thrombolysis, off-pump coronary artery bypass, percutaneous transluminal coronary angioplasty, extracorporeal circulation of cardiac surgery, cardiopulmonary-cerebral resuscitation, replantation of severed limbs, organ transplantation and the like.

10. The application according to claim 8, **characterized in that** the medicament is a combination of the traditional Chinese medicinal composition according to any one of claims 1-6 with tPA according to a ratio of 10: (5-20) during promotion of thrombolysis.
